# FASCICULE DE BREVET EUROPEEN

(11) **EP 4 329 614 B1**
(45) Date de publication et mention de la délivrance du brevet: **26.11.2025**
(21) Numéro de dépôt: 22725880.3
(22) Date de dépôt: 27.04.2022
(51) Int. Cl.: A61B 5/35, A61B 5/364, A61B 5/00, A61N 1/365, A61N 1/05

(54) **PROCÉDÉ D'ATTRIBUTION D'UNE INFORMATION D'IDENTIFICATION À UN SIGNAL D'UNE ÉLECTRODE CARDIAQUE**
VERFAHREN ZUR ZUORDNUNG EINER IDENTIFIKATIONSINFORMATION ZU EINEM SIGNAL EINER HERZELEKTRODE
PROCESS FOR ASSIGNING AN ITEM OF IDENTIFICATION INFORMATION TO A SIGNAL FROM A CARDIAC ELECTRODE

(30) Priorité: 27.04.2021 FR 2104376; 17.11.2021 FR 2112163
(43) Date de publication de la demande: 06.03.2024
(73) Titulaire: Centre Hospitalier Universitaire de Bordeaux, 33404 Talence Cedex (FR); UNIVERSITE DE BORDEAUX, 33000 Bordeaux (FR); FONDATION BORDEAUX UNIVERSITÉ, 33000 Bordeaux (FR)
(72) Inventeur: PLOUX, Sylvain, 33170 Gradignan (FR); STRIK, Marc, 33000 Bordeaux (FR); BORDACHAR, Pierre, 33000 Bordeaux (FR); DUBOIS, Rémi, 33700 Mérignac (FR)
(74) Mandataire: Oak & Fox
(86) Numéro de dépôt international: PCT/EP2022/061247
(87) Numéro de publication internationale: WO 2022/229281

(56) Documents cités:
- US-A1- 2017 042 482
- US-A1- 2017 128 734
- NAIR SANDEEP G ET AL: "Monitoring for and Diagnosis of Lead Dysfunction", CARDIAC ELECTROPHYSIOLOGY CLINICS, ELSEVIER, AMSTERDAM, NL, vol. 10, no. 4, 2 November 2018 (2018-11-02), pages 573 - 599, XP085532373, ISSN: 1877-9182, DOI: 10.1016/J.CCEP.2018.07.004

## Description

### Domaine de l'invention

Le domaine de l'invention se rapporte au domaine des procédés et des dispositifs de détection, d'analyse et de traitement de signaux cardiaques. En particulier, le domaine de l'invention se rapporte au domaine des procédés pour identifier des caractéristiques de signaux cardiaques issus d'une ou plusieurs sonde(s) de détection(s), telle qu'une ou plusieurs électrode(s) cardiaque(s), et pour attribuer des informations auxdits signaux.

### Etat de la technique

Il existe dans l'art antérieur des procédés appliqués aux stimulateurs cardiaques également connus dans la littérature sous le nom de « pacemaker » ou encore « pile cardiaque », et aux défibrillateurs tels que les défibrillateurs automatiques implantables, également désignés dans la littérature par l'acronyme « DAI », pour délivrer des chocs thérapeutiques à des patients.

De tels appareils comprennent des électrodes également appelées sondes qui permettent de détecter le rythme cardiaque, voire de délivrer des stimulations et/ou des chocs électriques dits « thérapeutiques » aux patients. Lorsqu'une modification du rythme est détectée, par exemple un rythme caractéristique d'une bradycardie, d'une fibrillation ventriculaire ou d'une tachycardie ventriculaire, une stimulation ou un choc électrique thérapeutiques peuvent être délivrés par l'appareil de sorte à rétablir un rythme cardiaque naturel.

Toutefois, un problème existe lorsqu'une sonde d'un stimulateur cardiaque ou d'un défibrillateur est endommagée. Lorsqu'une sonde est endommagée sur un « pacemaker », il existe un risque que le cœur du patient s'arrête et donc un risque de décès. Une sonde endommagée peut provenir, par exemple, de la rupture du câble interne de la sonde ou encore du fait que son isolant est ouvert.

Dans les deux cas, cela se manifeste par l'apparition de bruit, par intermittence ou de façon continue, sur un canal de détection de la sonde. Or, le bruit apparaissant sur le canal de détection peut être interprété à tort comme un signal d'origine cardiaque. Ce signal aura pour conséquence d'inhiber la stimulation sur un pacemaker avec un risque d'arrêt cardiaque et/ou d'entraîner la délivrance d'un choc électrique, voire de chocs électriques répétés, par un défibrillateur. Ces chocs électriques délivrés de manière inappropriée peuvent avoir des conséquences dramatiques sur les patients. Dès lors, il convient de mettre en place un procédé pour distinguer les signaux pathologiques des signaux dus à une électrode défectueuse.

**Il** existe, dans l'art antérieur, des procédés permettant de pallier ce problème de chocs électriques délivrés de manière inappropriée. À titre d'exemple, le brevet US7369893 décrit un procédé pour évaluer si la détection d'un signal par une sonde d'un stimulateur cardiaque est bien liée à un phénomène d'arythmie cardiaque et non à un phénomène de surdétection, en détectant une condition liée à la sonde. En effet, il est possible qu'une électrode soit défectueuse en raison de la rupture d'un conducteur et résulte en l'apparition de bruit sur le canal de détection. Toutefois, une telle méthode présente l'inconvénient de manquer de précision dans la détection du bruit dont l'apparition peut être due à des facteurs multiples.

D'autres documents pertinents de l'art antérieur sont US2017042482A1 et NAIR SANDEEP G ET AL : "Monitoring for and Diagnosis of Lead Dysfunction", CARDIAC ELECTROPHYSIOLOGY CLINICS, ELSEVIER, AMSTERDAM, NL, vol. 10, no. 4, 2 novembre 2018 (2018-11-02), pages 573-599, ISSN : 1877-9182, DOI : 10.1016/ J.CCEP.2018.07.004.

L'invention vise à exploiter une autre façon de déterminer la présence de bruit notamment en interprétant directement les signaux sur un canal de détection d'une sonde. En effet, en partant du principe avéré que le cœur d'un être humain ne peut se contracter plusieurs fois dans un intervalle de temps trop court, il est possible de constater sur le canal de détection d'une électrode qu'un signal ne peut pas être physiologique ; en comparant les intervalles temporels séparant plusieurs cycles cardiaques. Dès lors, l'apparition d'un signal non physiologique sur le canal de détection peut être attribuée à du bruit, et par conséquent, à une probabilité importante qu'une électrode soit défectueuse.

Un objectif de l'invention est de résoudre les inconvénients dus à l'apparition de bruit sur un canal de détection d'une électrode en proposant un procédé mis en œuvre par ordinateur pour attribuer une information d'identification à un signal issu de la détection d'un courant électrique cardiaque. Le procédé est basé sur les intervalles de temps séparant plusieurs signaux détectés, tels que les intervalles de temps entre plusieurs signaux successifs, et permet avantageusement de détecter avec précision l'apparition de bruit sur un canal de détection. Un autre avantage est de réduire le nombre d'alertes émises par les défibrillateurs et les « pacemakers » en réponse à la détection de faux positifs d'arythmies ventriculaires.

### Résumé de l'invention

L'invention est définie par les revendications annexées.

Selon un premier aspect, l'invention concerne un procédé mis en œuvre par ordinateur pour attribuer une information d'identification à un signal de détection, caractérisé en ce qu'il comprend :
- l'acquisition, au moyen d'une sonde, d'un premier signal de détection en réponse à la réception d'un courant électrique cardiaque, ledit premier signal de détection comportant une portion de signal correspondant à un motif électrique sur un premier intervalle;
- la mise en œuvre d'un algorithme de comparaison comprenant :
   ▪ la comparaison du premier intervalle avec un premier seuil ;
   ▪ la comparaison d'un second intervalle associé à une portion d'un second signal de détection antérieur au premier signal de détection avec un second seuil;
- l'attribution d'une information d'identification au premier signal de détection, ladite information d'identification étant déterminée en fonction du résultat des comparaisons réalisées par l'algorithme de comparaison, ladite information d'identification permettant de discriminer un signal de détection ayant une cause physiologique d'un signal de détection ayant pour cause un dysfonctionnement d'un matériel électronique.

Un avantage est de déterminer, sur la base des résultats des comparaisons mises en œuvre par l'algorithme, si un signal détecté correspond bien à un signal physiologique ; ou s'il s'agit d'un artefact de mesure potentiellement dû à une sonde cardiaque défectueuse. Un autre avantage est de détecter la présence de bruit par la mise en œuvre d'un algorithme peu complexe, et pouvant par conséquent être implémenté sur des appareils ne possédant que peu de puissance de calcul.

Selon un mode de réalisation, l'algorithme de comparaison comprend une étape de comparaison d'un troisième intervalle associé à une portion d'un troisième signal de détection avec un troisième seuil, ledit troisième signal de détection étant antérieur au second signal de détection.

Un avantage est d'améliorer l'identification de bruit sur les signaux détectés en ajoutant une étape de comparaison dans l'algorithme.

Selon un mode de réalisation, le procédé comprend la mise en œuvre d'un second algorithme de comparaison comprenant :
▪ Une étape de comparaison du premier intervalle avec un quatrième seuil ;
▪ Une étape de comparaison du second intervalle avec un cinquième seuil ;
▪ Une étape de comparaison du troisième intervalle avec un sixième seuil.

Un avantage est d'améliorer la détection du bruit sur un canal de détection d'une sonde ; par la mise en œuvre d'un autre algorithme comprenant des étapes de comparaison supplémentaires.

Selon un mode de réalisation, le procédé comprend la mise en œuvre, par le premier algorithme de comparaison ou le second algorithme de comparaison, de la comparaison d'un septième seuil avec un quatrième intervalle de temps associé à une portion d'un quatrième signal de détection antérieur au troisième signal de détection.

Un avantage est de permettre une distinction entre des signaux caractéristiques de phénomènes de surdétection et des signaux caractéristiques d'une rupture de sonde.

Selon un mode de réalisation, un ou plusieurs seuils parmi les premiers, second, troisième, quatrième, cinquième, sixième ou septième seuil sont définis par :
▪ Une valeur de seuil prédéfinie ou ;
▪ Une valeur médiane ou une moyenne de plusieurs valeurs entre elles ou ;
▪ Une fonction mathématique définie par rapport à un ou plusieurs intervalles de temps ou ;
▪ Une fonction mathématique indépendante des valeurs des intervalles acquis ou ;
▪ Une combinaison de plusieurs fonctions mathématiques.

Un avantage est de définir les critères d'attribution du type d'information d'identification à partir de seuils plus complexes pour obtenir une précision supplémentaire sur la détection de bruit sur le canal de détection.

Selon un mode de réalisation, l'information d'identification est attribuée au premier signal de détection en fonction du résultat des comparaisons d'au moins un des deux algorithmes de comparaison. Un avantage est d'exploiter les résultats des comparaisons d'au moins un des algorithmes pour identifier précisément le signal sur le canal de détection de l'électrode.

Selon un mode de réalisation, l'information d'identification attribuée au premier signal de détection comprend soit :
- une information physiologique définissant un rythme naturel de battement cardiaque ou une arythmie cardiaque ;
- une information d'anomalie caractérisant une donnée d'un signal non physiologique.

Un avantage est de caractériser l'information d'identification attribuée au signal de détection pour déterminer si ledit signal de détection correspond à la détection d'un rythme cardiaque ou si le signal de détection correspond à un artefact de mesure, par exemple dû au dysfonctionnement d'une électrode.

Selon un mode de réalisation, le procédé comprend :
- La génération d'une notification ou d'une alerte lorsque l'information d'identification attribuée au premier signal de détection comprend une information d'anomalie ;
- L'enregistrement de ladite alerte/notification au sein d'un espace mémoire,
- L'émission de ladite alerte/notification générée vers un équipement d'un réseau de données distant.

Un avantage est d'alerter le personnel compétent lorsqu'un défaut sur une électrode d'un patient est constaté.

Selon un mode de réalisation, l'information d'identification attribuée au premier signal de détection comprend une information d'anomalie soit lorsque :
- Le premier intervalle est inférieur au premier seuil et le second intervalle est supérieur au second seuil ;
- Le premier intervalle est inférieur au premier seuil et le second intervalle est supérieur au second seuil et le troisième intervalle est supérieur au troisième seuil.

Un avantage est de déduire la présence d'un signal non physiologique sur le canal de détection, sur la base de résultats de comparaisons entre des valeurs temporelles seuil caractéristiques et les intervalles associés aux signaux de détection.

Selon un mode de réalisation, le procédé comprend :
- la génération d'un électrogramme, ledit électrogramme comprenant une représentation graphique d'au moins un signal de détection parmi les signaux et d'au moins un intervalle ;
- l'acquisition d'au moins une image de l'électrogramme ;
- l'enregistrement de l'image de l'électrogramme acquise au sein d'un espace mémoire ;
- la transmission de l'image de l'électrogramme acquise vers au moins un équipement d'un réseau de données.

Un avantage est de pouvoir enregistrer et transmettre vers un équipement distant une représentation graphique des signaux détectés, qui pourront a postériori être examinés par un personnel compétent pour confirmer ou infirmer la présence de bruit sur le canal de détection.

Selon un mode de réalisation, le procédé comprend la génération d'une alerte visuelle, sonore et/ou par vibration lorsque l'information d'identification comprend une information d'anomalie.

Un avantage est d'alerter le patient de la présence d'une anomalie potentiellement due au dysfonctionnement d'un équipement.

Selon un mode de réalisation, l'information d'identification (lid) attribuée au premier signal de détection comprend une information d'anomalie caractéristique d'une rupture de sonde :
- Soit lorsque le premier intervalle de temps est inférieur au premier seuil, le second intervalle de temps est supérieur au second seuil et le troisième intervalle de temps est supérieur au troisième seuil ;
- Soit lorsque le premier intervalle de temps est inférieur au premier seuil et le second intervalle de temps est supérieur au second seuil, le troisième intervalle de temps est supérieur au troisième seuil et le quatrième intervalle de temps est supérieur au septième seuil.

Selon un autre aspect, l'invention concerne un système pour générer une information d'identification d'un signal de détection cardiaque comprenant :
- Un dispositif électrique comportant au moins une sonde pour acquérir un premier signal de détection en réponse à la réception d'un courant électrique cardiaque, ledit signal de détection comportant une portion de signal correspondant à un motif électrique sur un premier intervalle ;
- Un calculateur configuré pour mettre en œuvre :
   ▪ d'une part un algorithme de comparaison comprenant :
      i. Comparaison du premier intervalle avec un premier seuil;
      ii. Comparaison d'un second intervalle associé à une portion d'un second signal de détection antérieur au premier signal de détection avec un second seuil ;
- d'autre part une fonction d'attribution d'une information d'identification au premier signal de détection, ladite information d'identification étant déterminée en fonction du résultat des comparaisons réalisées par l'algorithme de comparaison, ladite information d'identification permettant de discriminer un signal de détection ayant une cause physiologique d'un signal de détection ayant pour cause un dysfonctionnement d'un matériel électronique.
- Un afficheur pour générer un marqueur graphique en superposition d'un électrogramme comportant le premier signal de détection afin de localiser temporellement l'information d'identification du premier signal de détection.
- Une mémoire pour enregistrer des données ;
- Une interface de communication pour échanger des données avec un équipement d'un réseau de données.

Selon un autre aspect, l'invention concerne un système configuré pour mettre en œuvre l'une quelconque des étapes du procédé selon l'invention, ledit système comprenant :
- le dispositif électrique comprenant au moins une sonde pour recevoir un courant électrique cardiaque ;
- un générateur de signaux pour générer le signal de détection en réponse à la réception du courant électrique cardiaque ;
- une mémoire pour enregistrer des données ;
- une interface de communication pour échanger des données avec un équipement d'un réseau de données distant ;

Selon un autre aspect, l'invention concerne un produit programme d'ordinateur comprenant des instructions qui, lorsque le programme est exécuté par un ordinateur, conduisent celui-ci à mettre en œuvre les étapes suivantes :
- Mise en œuvre d'un premier algorithme de comparaison comprenant :
   ▪ Comparaison d'un premier intervalle de temps, sur lequel s'étend un motif électrique d'une portion d'un premier signal de détection acquis au moyen d'une sonde, avec un premier seuil ;
   ▪ Comparaison d'un second intervalle de temps associé à une portion d'un second signal de détection antérieur au premier signal de détection avec un second seuil ;
- Attribution d'une information d'identification au premier signal de détection, ladite information d'identification étant déterminée en fonction du résultat des comparaisons réalisées par l'algorithme de comparaison et ladite information d'identification permettant de discriminer un signal de détection ayant une cause physiologique d'un signal de détection ayant pour cause un dysfonctionnement d'un matériel électronique.

### Brève description des figures

D'autres caractéristiques et avantages de l'invention ressortiront à la lecture de la description détaillée qui suit, en référence aux figures annexées, qui illustrent :
- Fig.1 : Un dispositif électrique comprenant une sonde implémentée dans le cœur d'un patient.
- Fig.2: Un logigramme de la mise en œuvre de l'algorithme de comparaison par un calculateur pour obtenir l'information d'identification.
- Fig.3 : Un logigramme de l'algorithme de comparaison mis en œuvre par un calculateur lorsqu'il comprend deux étapes de comparaison.
- Fig.4 : Un logigramme de l'algorithme de comparaison mis en œuvre par un calculateur lorsqu'il comprend trois étapes de comparaison.
- Fig.5 : Un logigramme de la mise en œuvre de deux algorithmes de comparaison comportant une pluralité d'étapes de comparaison.
- Fig.6 : Un visuel d'un électrogramme comprenant une représentation graphique de trois signaux de détection et des intervalles associés.
- Fig.7 : Un logigramme de la transmission d'une alerte à un équipement d'un réseau de données.
- Fig.8 : Un exemple de seuil défini par une fonction de deux intervalles de temps successifs.
- Fig.9 : un logigramme de la mise en œuvre de deux algorithmes de comparaison comportant une pluralité d'étapes de comparaison.
- Fig.10 : Un visuel d'un électrogramme comprenant une représentation graphique de quatre signaux de détection, et des intervalles associés.

### Description détaillée

Selon un premier aspect, l'invention concerne un procédé mis en œuvre par ordinateur pour attribuer une information d'identification l_{id} à un premier signal de détection S_{D}.

### Signaux de détection

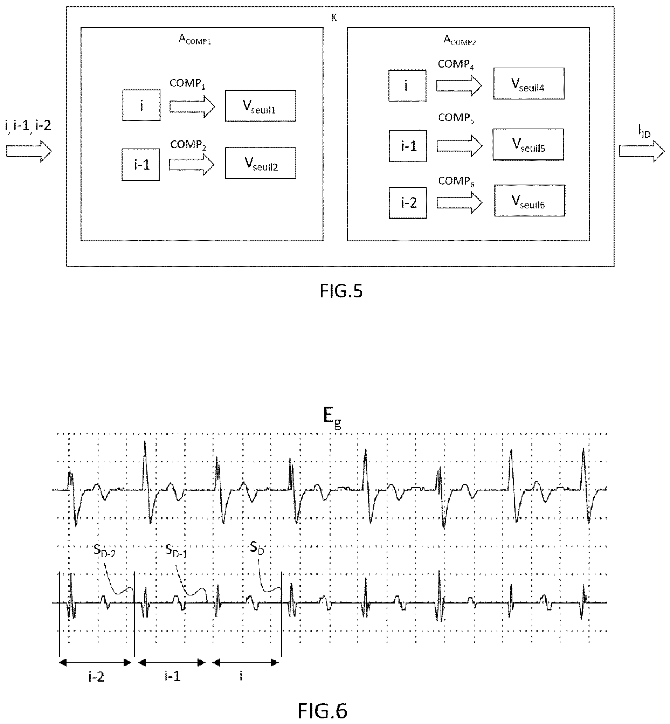

On entend par « signal de détection » une portion de signal caractéristique soit d'une activité électrique cardiaque, soit d'un défaut matériel, tel qu'une rupture de sonde, soit d'un ou plusieurs signaux extérieurs tels que des interférences électromagnétiques. Dans un exemple illustratif, en référence à la figure 6, une image d'un électrogramme E_{G} comprend une représentation graphique de plusieurs signaux de détection S_{D}, S_{D-1}, S_{D-2} représentant chacun une portion d'une activité électrique cardiaque en fonction du temps.

On nomme dans la suite de la description, un intervalle de temps la durée pendant laquelle est défini et acquis un signal de détection S_{D}. Ainsi, le premier signal de détection S_{D} est associé à un premier intervalle i. Le premier intervalle i correspond à un intervalle de temps. L'intervalle de temps i est défini entre deux évènements successifs. Les évènements sont, par exemple, de motifs, des patterns ou des signatures électriques reconnaissables ou caractéristiques d'une cause physiologique ou non physiologique.

La notion d'intervalle de temps i est donc liée à l'enchainement séquentiel des mesures d'un signal électrique d'une sonde positionnée au voisinage d'une source électrique produisant un signal régulier tel que peut l'être le cœur.

Les évènements peuvent correspondre à des singularités d'un motif électrique acquis tel que :
- Un maximum local ;
- Un minimum local ;
- La durée ou la présence de deux maximums locaux ou de minimums locaux ou une combinaison d'un maximum local et d'un minimum local ;
- D'une fréquence donnée ou d'un motif spectral donné, plus généralement d'un spectre ;
- Etc.

Selon un mode de réalisation, les évènements sont identifiés à postériori de l'acquisition d'un signal de détection SD, par exemple à partir d'au moins deux signaux S_{D-1} et S_{D} acquis pour en déduire une période ou une pseudo-période entre deux portions comparables de deux signaux de détection acquis successivement. Cette solution permet de garantir de considérer des évènements fiables pour en extraire un calcul d'intervalle.

Plus généralement, un évènement est défini pour constituer une référence de mesure d'intervalle.

Dans un exemple, le premier intervalle i comprend l'intervalle de temps séparant le premier signal de détection S_{D} d'un autre signal antérieur, par exemple le second signal de détection S_{D-1}. Par « antérieur », on entend que le second signal de détection S_{D-1} a été détecté par l'électrode avant le premier signal de détection S_{D}. Les deux signaux S_{D} et S_{D-1} sont par exemple détectés successivement. D'un point de vue physiologique, l'intervalle i correspond par exemple à l'intervalle de temps entre deux contractions cardiaques, telles que deux contractions ventriculaires.

Dans la suite de la description, les signaux de détection ayant pour cause un dysfonctionnement d'un matériel électronique sont également appelés des signaux de détection caractéristiques d'une activité non-physiologique.

Dans la suite de la description, on désignera indifféremment une électrode d'un dispositif électrique, tel que par exemple un défibrillateur, par les termes « électrode » ou « sonde ».

Parmi les signaux de détection caractéristiques d'une activité non-physiologique, on trouve par exemple et de manière non limitative :
- Des signaux générés par une rupture de sonde ;
- Des signaux générés par un défaut de connectique, par exemple au niveau du connecteur de la sonde dans le boîtier ;
- Des signaux issus d'un bruit de mesure ;
- Des signaux générés par une surdétection d'un signal physiologique au voisinage de la zone de mesure, ladite surdétection étant causée par une sur-sensibilité matérielle de la sonde au voisinage de la zone de mesure ;
- Des signaux générés par une sous-détection d'un signal physiologique au voisinage de la zone de mesure, ladite sous-détection étant causée par une sous-sensibilité matérielle de la sonde au voisinage de la zone de mesure,
- Des interférences de mesure produites par une combinaison de signaux entre des signaux caractéristiques d'une activité physiologique et de signaux non caractéristiques d'une activité physiologique.

Selon un mode de réalisation, le premier signal de détection S_{D} est acquis au moyen d'une électrode d'un dispositif électrique, tel que par exemple un défibrillateur automatique implantable.

Dans un exemple, en référence à la figure 6, le premier signal détecté par l'électrode suivant l'axe temporel est le troisième signal de détection S_{D-2} et le troisième signal détecté par l'électrode suivant l'axe temporel est le premier signal de détection S_{D}.

Selon un exemple, une pluralité de signaux de détection S_{D}, S_{D-1}, S_{D-2} sont acquis et correspondent chacun à un cycle caractéristique d'une activité électrique cardiaque. Ils sont par exemple acquis successivement en réponse à la détection d'un courant électrique par une sonde implémentée dans le ventricule droit du cœur d'un patient. Selon un cas, les signaux de détection S_{D}, S_{D-1}, S_{D-2} correspondent à trois contractions ventriculaires consécutives. Les signaux S_{D}, S_{D-1} et S_{D-2} correspondent par exemple à des signaux caractéristiques d'une tachycardie ventriculaire droite. Selon un autre exemple, le premier signal de détection S_{D} correspond à une donnée caractéristique d'un signal non physiologique.

### Algorithme de comparaison et information d'identification

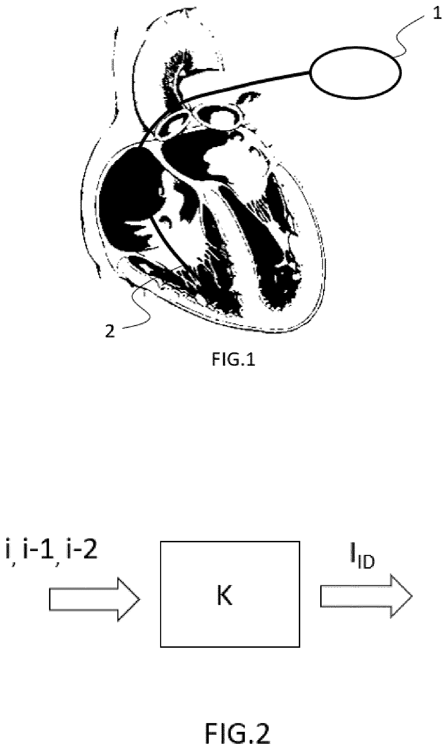

Dans un mode de réalisation, le procédé comprend la mise en œuvre d'un algorithme de comparaison A_{COMP1} des durées des intervalles des signaux acquis. Dans un exemple, en référence à la figure 2, l'algorithme de comparaison A_{COMP1} est mis en œuvre par un calculateur K. Le calculateur K est par exemple un calculateur compris par un dispositif médical tel qu'un défibrillateur automatique implantable (DAI).

Selon un cas, la comparaison de ces intervalles, entre eux ou avec des valeurs seuils, permet de déterminer si les signaux de détection S_{D}, S_{D-1}, S_{D-2} correspondent à une activité électrique cardiaque physiologique ou physiopathologique ou encore à un signal non physiologique.

À titre d'exemple, une succession de cycles courts peut correspondre à une réalité physiopathologique telle qu'une fibrillation ventriculaire ou une tachycardie ventriculaire. Selon un autre exemple, une alternance de cycles longs et de cycles courts ne correspond à aucune réalité physiologique et les signaux détectés sont associés à une information non physiologique, par exemple causée par un défaut matériel. On entend par « cycle court » un signal de détection comprenant un intervalle associé dont la valeur est inférieure à une valeur seuil prédéfinie, par exemple inférieure à 200 ms. On entend par « cycle long » un signal de détection comprenant un intervalle associé dont la valeur est supérieure à une valeur seuil prédéfinie, par exemple de 550 ms.

D'autres bornes ou valeurs seuils peuvent être définies dans une séquence d'un cycle long suivi d'un cycle court ou d'un cycle court suivi d'un cycle long. D'autres exemples sont ci-après détaillés selon les algorithmes de comparaison des intervalles mis en œuvre.

Selon la configuration de l'algorithme, la contrainte de seuil peut être implémentée sur le seuil du cycle long (seuil bas ou seuil élevé vis-à-vis d'une valeur moyenne) ou sur le seuil du cycle court (seuil bas ou élevé vis-à-vis d'une valeur moyenne). Selon un exemple, les intervalles de temps i, i-1, i-2 associés aux signaux de détection S_{D}, S_{D-1}, S_{D-2} sont des intervalles de temps mesurés en millisecondes. Toutefois, tout type d'unité de mesure temporelle adaptée est susceptible d'être utilisée selon les cas de mise en œuvre de l'invention.

### 1^{er} algorithme de comparaison

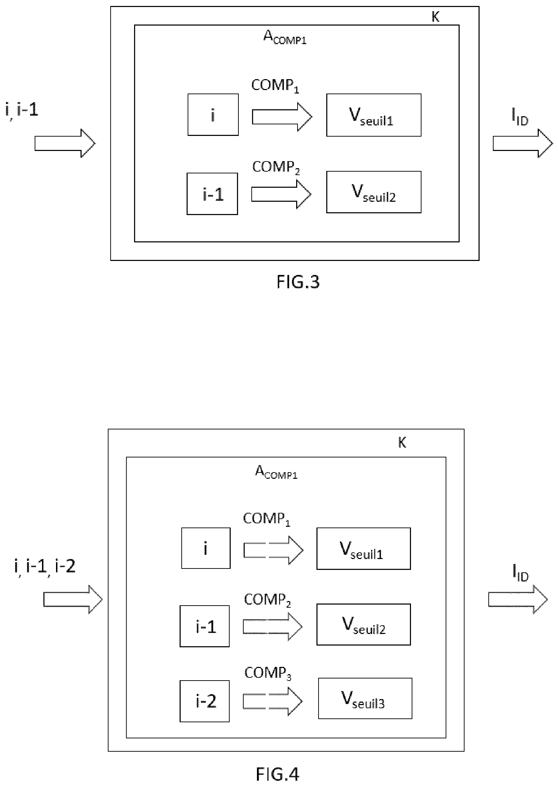

Dans un mode de réalisation, l'algorithme de comparaison A_{COMP1} comprend une pluralité d'étapes. En référence à la figure 3, la pluralité d'étapes comprend par exemple la mise en œuvre d'une première comparaison COMP₁ du premier intervalle i avec un premier seuil Vₛₑᵤᵢₗ₁ et la mise en œuvre d'une seconde comparaison COMP₂ du second intervalle i-1 avec un second seuil Vₛₑᵤᵢᵢ₂. On entend par « comparaison » d'un intervalle avec un seuil, le fait de déterminer si la valeur numérique associée audit intervalle est inférieure, supérieure ou encore égale audit seuil.

Dans un mode de réalisation en référence à la figure 4, la pluralité d'étapes mise en œuvre par l'algorithme de comparaison A_{COMP1} comprend la mise en œuvre d'une troisième comparaison COMP₃ du troisième intervalle i-2 avec un troisième seuil Vₛₑᵤᵢₗ₃. Un avantage est de faire varier les premiers et seconds seuils Vₛₑᵤᵢₗ₁, Vₛₑᵤᵢₗ₂, par exemple pour détecter le bruit sur un intervalle plus grand, par l'ajout d'un critère de comparaison supplémentaire.

Dans un exemple, les seuils Vₛₑᵤᵢₗ₁, Vₛₑᵤᵢₗ₂ de l'algorithme de comparaison A_{COMP1} lorsqu'il comprend deux étapes de comparaison COMP1, COMP₂ sont différents des seuils de ce même algorithme lorsqu'il comprend trois étapes de comparaison COMP₁, COMP₂, COMP₃. On pourra par exemple prendre des seuils Vₛₑᵤᵢₗ₁, Vₛₑᵤᵢₗ₂ plus grands dans la mise en œuvre de l'algorithme de comparaison A_{COMP1} lorsque celui-ci comprend trois étapes de comparaison. Cet exemple de mise en œuvre est particulièrement avantageux en ce qu'il permet de détecter une anomalie sur le signal de détection S_{D} sur une plage de valeurs plus large, par la mise en œuvre d'une troisième étape de comparaison.

Les seuils Vₛₑᵤᵢₗ₁, Vₛₑᵤᵢₗ₂ sont par exemple déterminées à l'issue de tests réalisés sur une pluralité de données pour déterminer des valeurs optimisées. Un avantage est d'identifier un maximum de signaux pouvant être associés à du bruit et donc potentiellement issus d'un défaut matériel, par exemple d'une sonde défectueuse.

Dans un mode de réalisation, les seuils Vₛₑᵤᵢₗ₁ et Vₛₑᵤᵢₗ₂ sont paramétrables par un utilisateur. Les seuils Vₛₑᵤᵢₗ₁, Vₛₑᵤᵢₗ₂ sont, par exemple, paramétrables au moyen d'une interface utilisateur. L'interface utilisateur est par exemple accessible sur un défibrillateur configuré pour mettre en œuvre le procédé selon l'invention. Selon un autre cas, les seuils Vₛₑᵤᵢₗ₁, Vₛₑᵤᵢₗ₂ sont paramétrables à partir d'un autre appareil, telle qu'une entité distante communiquant avec un dispositif médical configuré pour mettre en œuvre le procédé selon l'invention. Dans un exemple, les seuils Vₛₑᵤᵢₗ₁, Vₛₑᵤᵢₗ₂ sont paramétrés en amont de l'intégration de l'algorithme de comparaison A_{COMP1} dans un défibrillateur configuré pour mettre en œuvre le procédé de l'invention.

Selon un mode de réalisation, les seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ et Vₛₑᵤᵢₗ₇ peuvent prendre chacun n'importe quelle valeur en millisecondes notamment dans la liste de valeurs suivantes :
[5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, 500, 505, 510, 515, 520, 525, 530, 535, 540, 545, 550, 555, 560, 565, 570, 575, 580, 585, 590, 595, 600, 605, 610, 615, 620, 625, 630, 635, 640, 645, 650, 655, 660, 665, 670, 675, 680, 685, 690, 695, 700, 705, 710, 715, 720, 725, 730, 735, 740, 745, 750, 755, 760, 765, 770, 775, 780, 785, 790, 795, 800, 805, 810, 815, 820, 825, 830, 835, 840, 845, 850, 855, 860, 865, 870, 875, 880, 885, 890, 895, 900, 905, 910, 915, 920, 925, 930, 935, 940, 945, 950, 955, 960, 965, 970, 975, 980, 985, 990, 995]

Selon un mode de réalisation, les seuils Vₛₑᵤᵢₗ₁ et Vₛₑᵤᵢₗ₂ du premier algorithme de comparaison, lorsqu'il ne comprend que deux seuils, peuvent prendre n'importe quelle combinaison de valeurs en millisecondes parmi la liste de combinaisons de valeurs suivante :
[(5;360), (10;365), (15;370), (20;375), (25;380), (30;385), (35;390), (40;395), (45;400), (50;405), (55;410), (60;415), (65;420), (70;425), (75;430), (80;435), (85;440), (90;445), (95;450), (100;455), (105;460), (110;465), (115;470), (120;475), (125;480), (130;485), (135;490), (140;495), (145;500), (150;505), (155;510), (160;515), (165;520), (170;525), (175;530), (180;535), (185;540), (190;545), (195;550), (200;555), (205;560), (210;565), (215;570), (220;575), (225;580), (230;585), (235;590), (240;595), (245;600), (255;610), (265;620), (275;630), (285;640), (295;650), (305;660), (315;670), (325;680), (335;690), (345;700), (355;710), (365;720), (375;730), (385;740), (395;750), (405;760), (415;770), (425;780), (435;790), (445;800), (455;810), (465;820), (475;830), (485;840), (495;850), (505;860), (515;870), (525;880), (535;890), (545;900), (555;910), (565;920), (575;930), (585;940), (595;950), (605;960), (615;970), (625;980), (635;990)]

Selon un mode de réalisation, les seuils mis en œuvre dans l'un des algorithmes de comparaison A_{COMP1}, A_{COM2} lorsqu'ils comprennent 3 seuils, tels que les seuils Vₛₑᵤᵢₗ₁, Vₛₑᵤᵢₗ₂ et Vₛₑᵤᵢₗ₃ ou les seuils Vₛₑᵤᵢₗ₄, Vₛₑᵤᵢₗ₅ et Vₛₑᵤᵢₗ₆ peuvent prendre n'importe quelles valeurs parmi la liste de combinaisons de valeurs suivantes :
(40;250;250), (45;255;255), (50;260;260), (55;265;265), (60;270;270), (65;275;275), (70;280;280), (75;285;285), (80;290;290), (85;295;295), (90;300;300), (95;305;305), (100;310;310), (105;315;315), (110;320;320), (115;325;325), (120;330;330), (125;335;335), (130;340;340), (135;345;345), (140;350;350), (145;355;355), (150;360;360), (155;365;365), (160;370;370), (165;375;375), (170;380;380), (175;385;385), (180;390;390), (185;395;395), (190;400;400), (195;405;405), (200;410;410), (205;415;415), (210;420;420), (215;425;425), (220;430;430), (225;435;435), (230;440;440), (235;445;445), (240;450;450), (245;455;455), (250;460;460), (255;465;465), (260;470;470), (265;475;475), (270;480;480), (275;485;485), (280;490;490), (285;495;495), (290;500;500), (300;510;510), (310;520;520), (320;530;530), (330;540;540), (340;550;550), (350;560;560), (360;570;570), (370;580;580), (380;590;590), (390;600;600), (400;610;610), (410;620;620), (420;630;630), (430;640;640), (440;650;650), (450;660;660), (460;670;670), (470;680;680), (480;690;690), (490;700;700), (500;710;710), (510;720;720), (520;730;730), (530;740;740), (540;750;750), (550;760;760), (560;770;770), (570;780;780), (580;790;790), (590;800;800), (600;810;810), (610;820;820), (620;830;830), (630;840;840), (640;850;850), (650;860;860), (660;870;870), (670;880;880), (680;890;890), (690;900;900),

Dans cette version de l'algorithme, les seuils sont relevés et moins contraints, ce qui permet d'obtenir des informations qui n'auraient pas été détectées dans la mise en œuvre avec la première version de l'algorithme comportant deux seuils plus contraints (bornes plus basses et/ou plus hautes).

Toutefois, les exemples précités ne sont aucunement limitatifs et les seuils peuvent être paramétrés avec des valeurs différentes.

Selon différents modes de réalisation, les valeurs des différents seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ et Vₛₑᵤᵢₗ₇ sont compris dans des gammes de valeurs comprises entre 1 milliseconde et 1 seconde.

Selon un exemple dans lequel le premier algorithme de comparaison A_{COMP1} comprend uniquement les seuils Vₛₑᵤᵢₗ₁ et Vₛₑᵤᵢₗ₂, les seuils Vₛₑᵤᵢₗ₁ et Vₛₑᵤᵢₗ₂ sont par exemple compris entre 100 millisecondes et 700 millisecondes.

Selon un autre exemple, le seuil Vₛₑᵤᵢₗ₁ est compris entre 100 millisecondes et 300 millisecondes et le seuil Vₛₑᵤᵢₗ₂ est compris entre 400 millisecondes et 600 millisecondes lorsque le premier algorithme de comparaison A_{COMP1} comprend deux étapes de comparaison.

Selon un autre exemple, le seuil Vₛₑᵤᵢₗ₁ est compris entre 200 millisecondes et 300 millisecondes et les seuils Vₛₑᵤᵢₗ₂ et Vₛₑᵤᵢₗ₃ sont compris entre 400 millisecondes et 450 millisecondes lorsque le premier algorithme de comparaison A_{COMP1} comprend trois étapes de comparaison.

Selon un autre exemple, le seuil Vₛₑᵤᵢₗ₁ est compris entre 200 millisecondes et 250 millisecondes et les seuils Vₛₑᵤᵢₗ₂ et Vₛₑᵤᵢₗ₃ sont par exemple compris entre 400 millisecondes et 500 millisecondes lorsque le premier algorithme de comparaison A_{COMP1} comprend trois étapes de comparaison.

Selon un autre exemple, les seuils Vₛₑᵤᵢₗ₄, Vₛₑᵤᵢₗ₅ et Vₛₑᵤᵢₗ₆ du second algorithme de comparaison A_{COMP2} sont compris entre 200 millisecondes et 600 millisecondes.

Dans un autre exemple, le seuil Vₛₑᵤᵢₗ₄ est paramétré avec une valeur comprise entre 200 millisecondes et 300 millisecondes et les seuils Vₛₑᵤᵢₗ₅ et Vₛₑᵤᵢₗ₆ sont paramétrés avec des valeurs comprises entre 400 millisecondes et 500 millisecondes.

Dans un autre exemple, le seuil Vₛₑᵤᵢₗ₄ est paramétré avec une valeur comprise entre 200 millisecondes et 250 millisecondes et les seuils Vₛₑᵤᵢₗ5 et Vₛₑᵤᵢₗ₆ sont paramétrés avec des valeurs comprises entre 400 millisecondes et 450 millisecondes.

Selon un mode de réalisation, les seuils prédéfinis sont remplacés par des seuils dynamiques définis en fonction des intervalles de temps antérieur. Un exemple est la vérification et la détection d'un cycle long suivi d'un cycle court relativement à la durée de l'intervalle du cycle long. Le procédé permettrait par exemple de repérer un intervalle de temps du cycle court inférieur de 20% à l'intervalle du cycle long.

Selon différents cas, les valeurs choisies pour les seuils dans les algorithmes de comparaison A_{COMP1}, A_{COMP2} dépendent de la nature du patient. Par « nature » du patient, on entend que les seuils choisis seront par exemple différents en fonction de l'âge, du poids, du genre, des pathologies (cardiaques ou non), ou encore toute autre caractéristique du patient. Ainsi, les valeurs choisies pour les seuils dépendent au cas par cas de facteurs divers liés intrinsèquement au patient, de sorte à obtenir avantageusement une détection plus adaptée d'une information d'identification l_{id} caractéristique d'un signal non physiologique pour un patient donné.

### Combinaison du 1^{er} et du 2^{eme} algorithmes de comparaison

Dans un mode de réalisation, le procédé comprend la mise en œuvre d'un second algorithme de comparaison A_{COMP2}. Le second algorithme de comparaison A_{COMP2} comprend une pluralité d'étapes de comparaison. Selon un exemple, le second algorithme de comparaison A_{COMP2} est mis en œuvre consécutivement à la mise en œuvre du premier algorithme de comparaison A_{COMP1}. Dans un autre exemple, les deux algorithmes de comparaison A_{COMP1}, A_{COMP2} sont mis en œuvre parallèlement ou conjointement. Selon un autre cas, les deux algorithmes de comparaison A_{COMP1}, A_{COMP2} sont mis en œuvre indépendamment l'un de l'autre.

On rappelle que le premier algorithme peut comprendre deux ou trois seuils. Le second algorithme comprend au moins trois seuils. Lorsque les deux algorithmes sont mis en œuvre conjointement sur des mêmes mesures, ils sont alors préférentiellement configurés de sorte que le premier algorithme comporte deux seuils et le second algorithme comporte trois seuils.

Un avantage de cette configuration est d'obtenir des critères différents de classifications des anomalies temporelles des signaux détectés {surdétection, arythmie, artefact de mesure, etc.}. Ainsi, si un algorithme détecte un faux positif ou s'il rate une détection, la mise en œuvre du second algorithme permet d'augmenter la probabilité de détections des anomalies et de leur classification. Ainsi, un algorithme privilégie la définition de bornes temporelles contraintes (valeurs élevées ou basses) et un autre algorithme privilégie la persistance d'une anomalie sur plusieurs intervalles.

Dans un exemple illustré en figure 5, le premier algorithme de comparaison A_{COMP1} comprend la première comparaison COMP₁ du premier intervalle i avec le premier seuil Vₛₑᵤᵢₗ₁ et la seconde comparaison COMP₂ du second intervalle i-1 avec le second seuil Vₛₑᵤᵢₗ₂. Dans cet exemple, le second algorithme de comparaison A_{COMP2} comprend une quatrième comparaison du premier intervalle i avec un quatrième seuil Vₛₑᵤᵢₗ₄, une cinquième comparaison de second intervalle i-1 avec un cinquième seuil Vₛₑᵤᵢₗ₅ et une sixième comparaison du troisième intervalle i-2 avec un sixième seuil Vₛₑᵤᵢₗ₆. Dans cet exemple, la troisième comparaison COMP₃ n'est pas mise en œuvre et le premier algorithme A_{COMP1} ne comporte donc que deux étapes de comparaison.

Dans un mode de réalisation, les valeurs des seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ et Vₛₑᵤᵢₗ₇ sont interchangeables entre elles.

Dans un mode de réalisation, les algorithmes de comparaisons A_{COMP1}, A_{COMP2} sont paramétrables. Dans ce cas, un ou plusieurs opérateurs de comparaison mis en œuvre par lesdits algorithmes A_{COMP1}, A_{COMP2} sont par exemple susceptibles d'être modifiés.

### Seuils définis par une moyenne ou une médiane

Dans un mode de réalisation, au moins un seuil parmi les seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ et Vₛₑᵤᵢₗ₇ comprend une moyenne pondérée. La moyenne pondérée comprend par exemple une moyenne pondérée des intervalles i, i-1, i-2 et i-3 cycle à cycle. Un avantage est de d'identifier des événements particuliers, tel qu'un saut de fréquence ou une instabilité du système.

Dans un mode de réalisation, au moins un seuil parmi les seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ et Vₛₑᵤᵢₗ₇ comprend une valeur médiane. La valeur médiane comprend par exemple une valeur médiane des intervalles i, i-1, i-2 et i-3.

### Seuils définis par des fonctions

Dans un mode de réalisation, au moins un seuil parmi les seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ et Vₛₑᵤᵢₗ₇ comprend une fonction mathématique f. Selon un cas, la fonction mathématique f est une fonction qui dépend d'un intervalle de temps i, i-1, i-2 ou i-3, ou une fonction mathématique qui dépend de plusieurs de ces intervalles de temps. Selon d'autres cas, la fonction mathématique f dépend d'autres variables que les intervalles de temps i, i-1, i-2 ou i-3. La fonction mathématique f peut également comprendre une combinaison de plusieurs fonctions mathématiques entre elles.

Dans un autre exemple, la fonction mathématique f comprend une combinaison linéaire des intervalles de temps i, i-1 et i-2 tel que f = a(i) + b(i-1) + c(i-2) où a, b et c sont des constantes. Dans ce cas, l'intervalle i associé au signal de détection S_{D} est par exemple comparé avec la valeur prise par la fonction f en fonction des valeurs des intervalles de temps i, i-1 et i-2 pour déterminer si la valeur de l'intervalle de temps i est supérieure ou inférieure à la valeur prise par ladite fonction f. L'information d'identification l_{id} est ensuite attribuée au signal de détection S_{D} en fonction du résultat de la comparaison de l'intervalle i avec ladite fonction mathématique f.

Dans un autre exemple, la fonction mathématique f comprend une combinaison linéaire des intervalles de temps i, i-1, i-2 et i-3 telle que f = a(i) + b(i-1) + c(i-2) +d (i-3) où a, b, c et d sont des constantes.

Selon un mode de réalisation, plusieurs fonctions mathématiques f sont attribuées à différents seuils parmi les seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ et Vₛₑᵤᵢₗ₇. Ainsi, les intervalles de temps i, i-1, i-2 et i-3 sont comparés avec des fonctions mathématiques f différentes pour attribuer l'information d'identification l_{id} au signal de détection S_{D}.

Selon un mode de réalisation, au moins un des algorithmes de comparaison A_{COMP1}, A_{COMP2} comprend des seuils constants et des seuils variables. On entend par seuil « constant » l'attribution d'une valeur constante à un des seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ ou Vₛₑᵤᵢₗ₇. On entend par seuil « variable » l'attribution d'une fonction mathématique f à l'un des seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ ou Vₛₑᵤᵢₗ₇.

Dans un exemple, le premier algorithme de comparaison A_{COMP1} comprend le premier seuil Vₛₑᵤᵢₗ₁ auquel est attribuée une fonction mathématique f(i, i-1) et le second seuil Vₛₑᵤᵢₗ₂ auquel est attribué une valeur constante, par exemple 500ms. Ainsi, le premier intervalle de temps i est comparé avec la fonction mathématique f qui dépend des premiers et seconds intervalles de temps i, i-1 et le second intervalle de temps est comparé avec une valeur constante. L'information d'identification l_{id} est ensuite attribuée au signal de détection S_{D} en fonction du résultat de ces comparaisons.

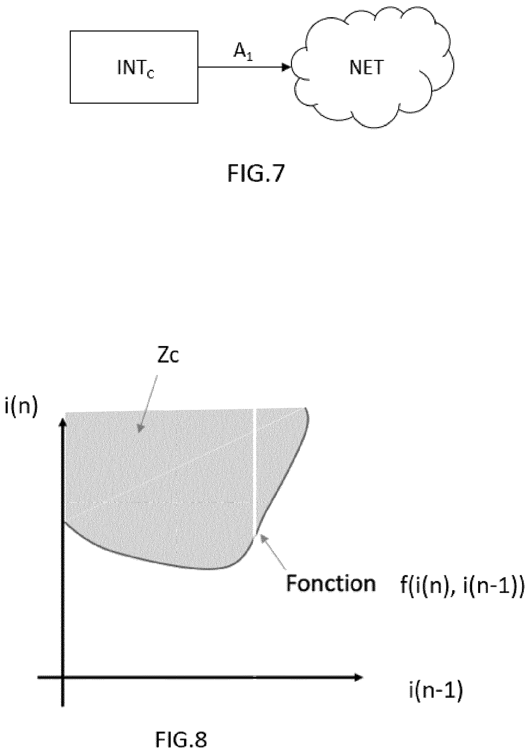

Selon un cas, en référence à la figure 8, au moins un des deux algorithmes de comparaison A_{COMP1}, A_{COMP2} comprend une zone de déclenchement Z_{C}. Le type d'information d'identification l_{id} attribuée au signal de détection S_{D} dépend du fait que le résultat des différentes comparaisons mises en œuvre par l'un des algorithmes A_{COMP1}, A_{COMP2} corroborent avec la zone de déclenchement Z_{C} d'un des algorithmes.

A titre d'exemple, dans le cas où les seuils Vₛₑᵤᵢₗ₁ et Vₛₑᵤᵢₗ₂ du premier algorithme de comparaison A_{COMP1} sont des constantes, la zone de déclenchement Z_{C} correspond par exemple à la zone dans laquelle le premier intervalle de temps i est inférieur au premier seuil Vₛₑᵤᵢₗ₁ et le second intervalle de temps i-1 est supérieur au second seuil Vₛₑᵤᵢₗ₂.

Ainsi, la zone de déclenchement Z_{C} représentée par une zone graphique sur la figure 8 comprend l'ensemble des points correspondant aux cas pour lesquels les critères de l'un des algorithmes de comparaison A_{COMP1}, A_{COMP2} sont vérifiés et pour lesquels une information d'identification l_{id} spécifique est attribuée au signal de détection S_{D}, par exemple une information d'anomalie caractéristique d'un signal non physiologique.

Le procédé comprend l'attribution d'une information d'identification l_{id} au premier signal de détection S_{D}. L'information d'identification l_{id} comprend par exemple une information sur la nature du signal de détection S_{D}. On entend par « nature du signal de détection S_{D} » le fait qu'un signal de détection S_{D} corresponde à une réalité physiologique ou physiopathologique ou encore que le signal de détection S_{D} n'ait aucune réalité physiologique ou physiopathologique et soit par exemple du bruit.

Dans un mode de réalisation, l'information d'identification l_{id} comprend une information physiologique. Selon un exemple, l'information physiologique comprend une information sur un rythme de battement naturel du cœur.

Dans un mode de réalisation, l'information d'identification l_{id} comprend une information physiopathologique. Selon divers exemples, l'information physiopathologique comprend une information sur une arythmie cardiaque telle qu'une bradycardie, une tachycardie ou encore une fibrillation. Cette information correspond par exemple à une arythmie détectée par une sonde positionnée dans une cavité cardiaque d'un patient, telle que le ventricule droit.

Toutefois, le type d'information d'identification l_{id} associée au signal de détection S_{D} ne se limite pas aux exemples précités et peut comprendre tout type d'information associée à un rythme cardiaque particulier identifié et associé au premier signal de détection S_{D}.

Dans un mode de réalisation, l'information d'identification l_{id} comprend une information d'anomalie. Selon un cas, l'information d'anomalie caractérise un signal non physiologique, par exemple issu du dysfonctionnement d'une électrode. Dans un exemple, l'information d'anomalie est associée au premier signal de détection S_{D} lorsque ledit premier signal de détection S_{D} est généré par un dispositif comportant une électrode défectueuse et se manifeste sous forme de bruit sur le canal de détection.

### Phénomène de surdétection et troisième critère

Dans un mode de réalisation, l'information d'anomalie comprend une information de « surdétection ». On entend par « surdétection » la détection d'un signal ayant une origine à la fois différente du signal S_{D} dû à la contraction d'une cavité dans laquelle la sonde est implémentée, et à la fois différente d'un signal caractéristique d'une information non physiologique, tel qu'un signal dû à une rupture de sonde. Selon différents exemples, l'information de « surdétection » comprend une information sur la détection d'une activité électrique cardiaque issue d'une décontraction ventriculaire, ou encore issue de la contraction de l'oreillette, lorsque la sonde est positionnée dans le ventricule droit. Plus généralement, l'information de « surdétection » est attribuée au premier signal de détection S_{D} lorsque ce dernier ne résulte pas d'une contraction de la cavité dans laquelle la sonde est implémentée et que le premier signal de détection S_{D} n'est pas caractéristique d'un signal non physiologique, par exemple résultant d'un défaut matériel.

Dans une variante, l'information d'anomalie comprend une information de « sous-détection ». On entend par « sous-détection » qu'une partie de l'activité électrique cardiaque n'a pas été correctement détectée par la sonde.

Selon un mode de réalisation, l'algorithme de comparaison A_{COMP2} comprend la mise en œuvre d'une septième étape de comparaison COMP₇.

Dans un mode de réalisation, l'algorithme de comparaison A_{COMP1} comprend la mise en œuvre de la septième étape de comparaison COMP₇.

La septième étape de comparaison COMP₇ comprend par exemple la comparaison du quatrième intervalle de temps i-3 avec un septième seuil Vₛₑᵤᵢₗ₇. Dans ce cas, le second algorithme A_{COMP2} comprend alors quatre seuils de comparaison. Lorsque le premier algorithme de comparaison comprend la mise en œuvre de la septième étape de comparaison COMP7, il comprend alors trois ou quatre seuils de comparaison. Cette mise en œuvre est particulièrement avantageuse pour distinguer les signaux caractéristiques des problèmes de rupture de sondes des signaux caractéristiques des phénomènes de surdétection. En effet, les phénomènes de surdétection non liés à des ruptures de sondes sont pour la plupart caractérisés par un seul cycle dit « court », c'est-à-dire inférieur à un intervalle de temps prédéfini. Dès lors, pour s'assurer que le phénomène détecté est bien dû à une rupture de sonde, il convient de s'assurer que plusieurs cycles courts se succèdent. En d'autres termes, on cherche à s'assurer qu'un intervalle succédant à un intervalle donné inférieur à un seuil prédéfini soit lui aussi inférieur à un seuil prédéfini (par exemple le même seuil).

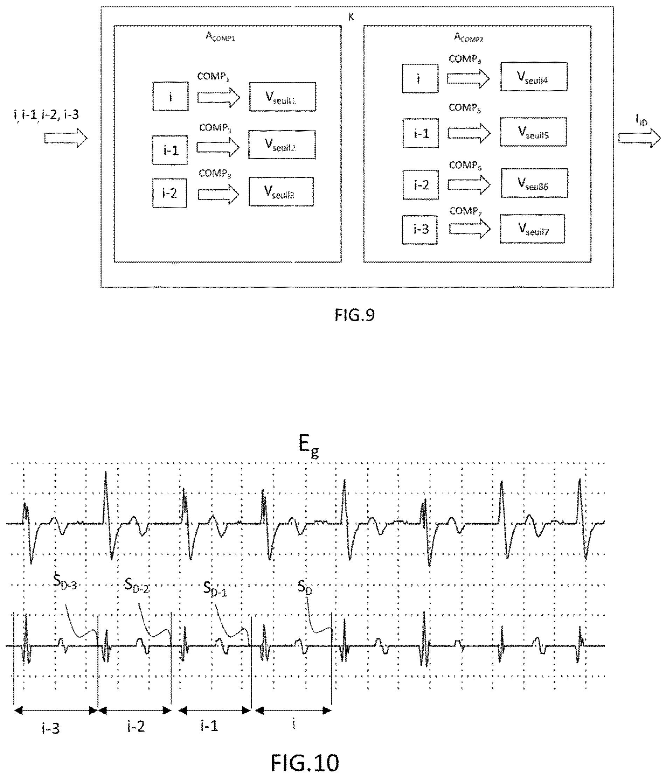

Pour illustrer ce mode, on se place dans le cas d'exemple illustré en figure 9 où le premier algorithme de comparaison A_{COMP1} comprend trois étapes de comparaisons COMP₁, COMP₂ et COMP₃ et que le second algorithme de comparaison A_{COMP2} comprend quatre étapes de comparaison COMP₄, COMP₅, COMP₆ et COMP₇. Dans ce cas, les comparaisons COMP₁, COMP₂ pour le premier algorithme A_{COMP1} et les comparaisons COMP₄, COMP₅ pour le second algorithme sont mises en œuvre pour s'assurer que les intervalles de temps successifs i et i-1 sont chacun inférieurs à des seuils prédéfinis (et donc caractéristiques de cycles courts). Dès lors, la mise en œuvre de l'algorithme permet à la fois d'optimiser la détection de bruit par la mise en œuvre de deux algorithmes successivement prenant en compte plusieurs critères de comparaison, et également de s'assurer que le bruit détecté est bien issu d'une rupture de sonde et non pas d'un phénomène de surdétection.

Selon un autre exemple de réalisation, le premier algorithme de comparaison A_{COMP1} ne comprend que deux étapes de comparaison COMP₁, COMP₂ et le second algorithme de comparaison A_{COMP2} comprend quatre étapes de comparaison COMP₄, COMP₅, COMP₆, COMP₇. Dans ce cas, la distinction entre le phénomène de surdétection et le problème de rupture de sonde est étudiée uniquement via le second algorithme de comparaison A_{COMP2}. Dans cet exemple, on vérifie que deux cycles courts successifs succèdent à deux cycles longs successifs pour déterminer un problème de rupture de sonde.

Selon un autre exemple de réalisation, le premier algorithme de comparaison A_{COMP1} comprend trois étapes de comparaison COMP₁, COMP₂, COMP₃ et le second algorithme de comparaison A_{COMP2} comprend trois étapes de comparaison COMP₄, COMP₅, COMP₆. Dans ce cas, la distinction entre la surdétection et la rupture de sonde est par exemple uniquement étudiée au travers d'un algorithme parmi les premiers et seconds algorithmes de comparaison A_{COMP1}, A_{COMP2}. Dans ce cas, on vérifie par exemple qu'un cycle long est suivi de deux cycles courts successifs.

Selon un mode de réalisation, le premier algorithme de comparaison A_{COMP1} comprend deux étapes de comparaison COMP₁, COMP₂ et le second algorithme de comparaison A_{COMP2} comprend trois étapes de comparaison COMP₄, COMP₅, COMP₆. Dans ce cas, la distinction entre le phénomène de surdétection et le problème de rupture de sonde est par exemple uniquement étudiée via le second algorithme de comparaison A_{COMP2}.

Selon un mode de réalisation, les seuils mis en œuvre dans l'algorithme de comparaison A_{COMP1} ou l'algorithme de comparaison A_{COMP2} lorsqu'ils comprennent 4 seuils, tels que les seuils Vₛₑᵤᵢₗ₄, Vₛₑᵤᵢₗ₅, Vₛₑᵤᵢₗ₆ et Vₛₑᵤᵢₗ₇ peuvent prendre n'importe quelles valeurs parmi la liste de combinaisons de valeurs suivantes :

| | | |
|---|---|---|
| (40;40;250;250), | (45;45;255;255), (50;50;260;260), | (55;55;265;265), |
| (60;60;270;270), | (65;65;275;275), (70;70;280;280), | (75;75;285;285), |
| (80;80;290;290), | (85;85;295;295), (90;90;300;300), | (95;95;305;305), |
| (100;100; 310;310), | (105;105;315;315), (110;110;320;320), | (115;115;325;325), |
| (120;120;330;330), | (125;125;335;335), | (130;130;340;340), |
| (135;135;345;345), | (140;140;350;350), | (145;145;355;355), |
| (150;150;360;360), | (155;155;365;365), | (160;160;370;370), |
| (165;165;375;375), | (170;170;380;380), | (175;175;385;385), |
| (180;180;390;390), | (185;185;395;395), | (190;190;400;400), |
| (195;195;405;405), | (200;200;410;410), | (205;205;415;415), |
| (210;210;420;420), | (215;215;425;425), | (220;220;430;430), |
| (225;225;435;435), | (230;230;440;440), | (235;235;445;445), |
| (240;240;450;450), | (245;245;455;455), | (250;250;460;460), |
| (255;255;465;465), | (260;260;470;470), | (265;265;475;475), |
| (270;270;480;480), | (275;275;485;485), | (280;280;490;490), |
| (285;285;495;495), | (290;290;500;500), | (300;300;510;510), |
| (310;310;520;520), | (320;320;530;530), | (330;330;540;540), |
| (340;340;550;550), | (350;350;560;560), | (360;360;570;570), |
| (370; 370; 580; 580), | (380;380;590;590), | (390;390;600;600), |
| (400;400;610;610), | (410;410;620;620), | (420;420;630;630), |
| (430;430;640;640), | (440;440;650;650), | (450;450;660;660), |
| (460;460;670;670), | (470;470;680;680), | (480;480;690;690), |
| (490;490;700;700), | (500;500;710;710), | (510;510;720;720), |
| (520;520;730;730), | (530;530;740;740), | (540;540;750;750), |
| (550;550;760;760), | (560;560;770;770), | (570;570;780;780), |
| (580;580;790;790), | (590;590;800;800), | (600;600;810;810), |
| (610;610;820;820), | (620;620;830;830), | (630;630;840;840), |
| (640;640;850;850), | (650;650;860;860), | (660;660;870;870), |
| (670;670;880;880), | (680;680;890;890), (690;690;900;900). | |

Dans cette version de l'algorithme, l'introduction d'un seuil supplémentaire permet avantageusement de distinguer les signaux dus à un phénomène de surdétection des signaux dus à un problème de rupture de sonde. Par exemple, une succession de deux cycles courts précédée d'une succession de deux cycles longs peut correspondre à une rupture de sonde.

Selon un mode de réalisation, les seuils mis en œuvre dans l'un des algorithmes de comparaison A_{COMP1}, A_{COM2} lorsqu'ils comprennent 3 seuils, tels que les seuils Vₛₑᵤᵢₗ₁, Vₛₑᵤᵢₗ₂ et Vₛₑᵤᵢₗ₃ ou les seuils Vₛₑᵤᵢₗ₄, Vₛₑᵤᵢₗ₅ et Vₛₑᵤᵢₗ₆ peuvent prendre n'importe quelles valeurs parmi la liste de combinaisons de valeurs suivantes :

| | | | | |
|---|---|---|---|---|
| (40;40;250), | (45;45;255), | (50;50;260), (55;55;265), | (60;60;270), | (65;65;275), |
| (70;70;280), | (75;75;285), | (80;80;290), (85;85;295), | (90;90;300), | (95;95;305), |
| (100;100;310), | (105;105;315), | (110;110;320), | (115;115;325), | (120;120;330), |
| (125;125;335), | (130;130;340), | (135;135;345), | (140;140;350), | (145;145;355), |
| (150;150;360), | (155;155;365), | (160;160;370), | (165;165;375), | (170;170;380), |
| (175;175;385), | (180;180;390), | (185;185;395), | (190;190;400), | (195;195;405), |
| (200;200;410), | (205;205;415), | (210;210;420), | (215;215;425), | (220;220;430), |
| (225;225;435), | (230;230;440), | (235;235;445), | (240;240;450), | (245;245;455), |
| (250;250;460), | (255;255;465), | (260;260;470), | (265;265;475), | (270;270;480), |
| (275;275;485), | (280;280;490), | (285;285;495), | (290;290;500), | (300;300;510), |
| (310;310;520), | (320;320;530), | (330;330;540), | (340;340;550), | (350;350;560), |
| (360;360;570), | (370;370;580), | (380;380;590), | (390;390;600), | (400;400;610), |
| (410;410;620), | (420;420;630), | (430;430;640), | (440;440;650), | (450;450;660), |
| (460;460;670), | (470;470;680), | (480;480;690), | (490;490;700), | (500;500;710), |
| (510;510;720), | (520;520;730), | (530;530;740), | (540;540;750), | (550;550;760), |
| (560;560;770), | (570;570;780), | (580;580;790), | (590;590;800), | (600;600;810), |
| (610;610;820), | (620;620;830), | (630;630;840), | (640;640;850), | (650;650;860), |
| (660;660;870), | (670;670;880), | (680;680;890), | (690;690;900), | |

Un avantage d'utiliser les valeurs de seuils précitées dans l'un ou l'autre des algorithmes de comparaison A_{COMP1}, A_{COMP2} est de conserver une bonne sensibilité de détection de rupture de sonde tout en améliorant la distinction entre les phénomènes de surdétection et les problèmes de rupture de sonde.

Dans le cas où la sensibilité des ruptures de sonde serait affectée par la mise en place d'un nouveau critère, cette sensibilité tendra à s'améliorer au cours du temps puisque davantage de bruit sera détecté ; et que par conséquent, la probabilité de détection de succession d'intervalles courts augmentera.

Dans un mode de réalisation, le type d'information d'identification l_{id} associée au signal de détection S_{D} dépend du résultat d'une ou plusieurs comparaisons réalisées par l'algorithme de comparaison A_{COMP1}.

Selon un exemple, le premier intervalle i est inférieur au premier seuil Vₛₑᵤᵢₗ₁ et le second intervalle i-1 est inférieur au second seuil Vₛₑᵤᵢₗ₂. Le premier algorithme de comparaison A_{COMP1} a par exemple été paramétré de sorte que, si les critères des comparaisons des intervalles i, i-1 avec lesdits seuils Vₛₑᵤᵢₗ₁, Vₛₑᵤᵢₗ₂ ne sont pas remplis, c'est-à-dire que le premier intervalle i est supérieur à la valeur seuil Vₛₑᵤᵢₗ₁ ou que le second intervalle i-1 est inférieur à la seconde valeur seuil Vₛₑᵤᵢₗ₂, le premier signal de détection S_{D} ne peut pas avoir de réalité physiologique ou physiopathologique. Dès lors, une information d'identification l_{id} comprenant une information d'anomalie est attribuée au premier signal de détection S_{D}.

Selon un autre exemple illustratif, le premier intervalle i est inférieur au premier seuil Vₛₑᵤᵢₗ₁ et le second intervalle i-1 est supérieur au second seuil Vₛₑᵤᵢₗ₂. Le premier algorithme de comparaison A_{COMP1} a par exemple été paramétré de sorte que, si les critères des comparaisons des intervalles i, i-1 avec lesdits seuils Vₛₑᵤᵢₗ₁, Vₛₑᵤᵢₗ₂ sont remplis, c'est-à-dire que le premier intervalle i est inférieur à la valeur seuil Vₛₑᵤᵢₗ₁ ou que le second intervalle i-1 est supérieur à la seconde valeur seuil Vₛₑᵤᵢₗ₂, le premier signal de détection S_{D} ne peut pas avoir de réalité physiologique ou physiopathologique. Dès lors, une information d'identification l_{id} comprenant une information d'anomalie est attribuée au premier signal de détection S_{D}.

Dans un mode de réalisation, le type d'information d'identification l_{id} attribuée au premier signal de détection S_{D} dépend du résultat des comparaisons mises en œuvre par au moins un des deux algorithmes de comparaison A_{COMP1}, A_{COMP2}. Avantageusement, le fait que les conditions d'un seul des deux algorithmes de comparaison A_{COMP1}, A_{COMP2} ne soient pas remplies entraîne l'attribution d'une information d'anomalie au premier signal de détection S_{D}. Un autre avantage est de rendre plus performante la détection du bruit sur le canal de détection ; par la mise en place d'un nombre plus élevé de critères de comparaison.

Dans un mode de réalisation, l'information d'identification (l_{id}) est stockée dans un espace mémoire. L'espace mémoire comprend par exemple un espace de stockage. L'espace mémoire est par exemple implémenté sur un dispositif médical, tel qu'un défibrillateur automatique implantable.

Dans un mode de réalisation, le procédé comprend la génération d'une notification ou d'une alerte A₁ lorsque l'information d'identification l_{id} comprend une information d'anomalie. Selon un cas, l'alerte A₁ est enregistrée au sein de l'espace mémoire.

Dans un mode de réalisation, la notification ou alerte A₁ est générée à la suite de l'attribution de plusieurs informations d'identifications l_{id} comprenant chacune une information d'anomalie. Selon un exemple, l'alerte ou notification A₁ est transmise lorsque cinq informations d'identifications l_{id} comprennent une information d'anomalie. Un avantage est de restreindre l'émission de l'alerte à la détection répétée d'événements non physiologiques. Cela permet par exemple d'éviter les « faux-positifs » de d'évènements non physiologiques et donc d'éviter une remontée d'alerte trop fréquente ou inadaptée.

Dans un mode de réalisation, en référence à la figure 7, l'alerte A₁ est transmise vers un équipement d'un réseau de données NET. L'alerte A₁ est par exemple transmise au moyen d'un signal émis par une interface de communication INT_{C}. L'équipement d'un réseau de données NET comprend par exemple un serveur distant. Dans un exemple, l'alerte A₁ est transmise via une alerte de télémédecine. Avantageusement, l'alerte A₁ ainsi transmise peut ensuite être lue par un utilisateur, par exemple un personnel médical, pour constater le dysfonctionnement de l'appareil d'un patient et agir en conséquence. Les risques de décès d'un patient liés au dysfonctionnement d'une électrode sont avantageusement réduits.

### Seuils en cascade pour les algorithmes

Dans un mode de réalisation, au moins un des deux algorithmes de comparaison A_{COMP1}, A_{COMP2} comprend d'autres étapes de comparaison que les étapes COMP₁, COMP₂, COMP₃, COMP₄, COMP₅, COMP₆, COMP₇.

Ainsi, la mise en œuvre d'un ou de deux algorithmes comprenant des étapes de comparaisons d'intervalles de temps associés à des signaux de détection rentre dans le cadre de l'invention ; quel que soit le nombre d'étapes de comparaisons mises en œuvre par les algorithmes.

### Analyse cycle à cycle en continu

Dans un mode de réalisation, le procédé comprend l'acquisition d'une pluralité de signaux de détection S_{D} en continu. On rappelle qu'un intervalle de temps est associé à chaque nouveau signal de détection S_{D} acquis. Cet intervalle de temps correspond donc à l'intervalle de temps le séparant d'un signal antérieur, par exemple un signal S_{D-1} le précédant.

Dans ce processus continu d'analyse, le procédé de l'invention est réalisé de manière continue en considérant des intervalles successifs en « glissant » les intervalles comparés tout au long de l'analyse.

Ainsi, pour chaque signal de détection S_{D} acquis, une comparaison de l'intervalle de temps i qui lui est associé avec des intervalles de temps associés à des signaux antérieurs, par exemple au moyen de l'algorithme de comparaison A_{COMP1}, permet d'obtenir une information d'identification I_{ID} dudit signal de détection S_{D}. Dès lors, la comparaison en continu des intervalles associés à chaque nouveau signal de détection acquis et des intervalles associés à des signaux antérieurs, par exemple avec des seuils au moyen de l'un des algorithmes de comparaison A_{COMP1}, A_{COMP2}, permet d'obtenir une information d'identification I_{ID} pour chaque nouveau signal de détection acquis S_{D}.

Ce mode est particulièrement avantageux pour déterminer un taux d'incidence du bruit sur le canal de détection, par exemple en observant une fréquence d'apparition d'une information d'anomalie associée à chaque information d'identification I_{id}. Ainsi cette analyse cycle à cycle permet d'extraire une information spectrale liée d'une part au rythme d'apparition des informations d'identification produites par le procédé de l'invention et d'autre part au rythme d'apparition des anomalies d'arythmie causées par des signaux physiologiques. Cette analyse spectrale permet de corroborer des informations et donc par exemple de confirmer un défaut matériel.

### Electrogramme

Dans un mode de réalisation, le procédé de l'invention comprend la génération d'un électrogramme E_{G}. L'électrogramme E_{G} comprend par exemple un enregistrement de l'activité cardiaque d'un patient sur un intervalle de temps donné.

Selon un mode de réalisation, en référence à la figure 6, l'électrogramme E_{G} comprend une représentation graphique des trois signaux de détection S_{D}, S_{D-1}, S_{D-2} et des intervalles de temps i, i-1 et i-2 associés auxdits signaux de détection.

Selon un autre mode de réalisation illustré en figure 10, l'électrogramme E_{G} comprend une représentation graphique de quatre signaux de détection S_{D}, S_{D-1}, S_{D-2}, S_{D-3} et des intervalles de temps i, i-1, i-2, i-3 associés auxdits signaux de détection.

Dans un mode de réalisation, le procédé comprend l'acquisition d'une image de l'électrogramme E_{G}. Selon un cas, l'acquisition de l'image de l'électrogramme E_{G} a lieu lorsque l'information d'identification I_{id} comprend une information d'anomalie. Selon un autre cas, l'acquisition d'une image de l'électrogramme E_{G} a lieu périodiquement, par exemple suivant une période prédéfinie. Dans un exemple, l'image de l'électrogramme E_{G} est enregistrée au sein d'un espace mémoire.

Selon un mode de réalisation, l'image de l'électrogramme E_{G} est transmise vers un équipement d'un réseau de données NET. L'image de l'électrogramme E_{G} est par exemple transmise par exemple au moyen de l'interface de communication INT_{C}. L'image de l'électrogramme E_{G} est par exemple automatiquement transmise suite à son enregistrement au sein de l'espace mémoire.

Dans un mode de réalisation, l'image de l'électrogramme E_{G} est transmise en même temps que l'alerte A_{1.} L'image de l'électrogramme E_{G} est par exemple via une alerte de télémédecine. Un avantage est de permettre au personnel compétent de prendre connaissance de la représentation graphique des signaux pour pouvoir, a posteriori, confirmer ou infirmer la présence de bruit sur le canal de détection de la sonde.

Dans un mode de réalisation, la transmission de l'image de l'électrogramme E_{G} vers un équipement d'un réseau de données NET entraîne automatiquement sa suppression de l'espace mémoire sur lequel ladite image est stockée. Un avantage est de libérer de l'espace mémoire lorsque l'image de l'électrogramme E_{G} a été transmise au personnel médical compétent pour être analysée. Selon un autre cas, les images enregistrées sont supprimées périodiquement de l'espace mémoire, selon une période temporelle prédéfinie.

Dans un mode de réalisation, le procédé comprend la désactivation d'au moins un algorithme de comparaison parmi les algorithmes A_{COMP1}, A_{COMP2}. Dans un exemple, la désactivation de l'algorithme est effective pendant une première période temporelle La désactivation de l'algorithme de comparaison A_{COMP1} est par exemple mise en œuvre consécutivement à la réception d'une commande de désactivation. Selon un cas, la commande de désactivation reçue a été préalablement émise par une entité distante.

Dans un exemple de réalisation, l'alerte A₁ et l'électrogramme E_{G} sont transmis via une alerte de télémédecine vers une entité distante telle qu'un équipement d'un réseau de données NET. Les signaux de l'électrogramme E_{G} sont par exemple analysés par un personnel médical pour déterminer si le signal de détection S_{D} correspond bien à la détection d'un signal caractéristique d'une activité électrique non physiologique. Une commande de désactivation de l'algorithme de comparaison est ensuite reçue si l'alerte A₁ émise est due à un faux positif. Un avantage est d'éviter une remontée d'alertes trop fréquente due à des faux positifs.

### Dispositif électrique (stimulateur cardiaque et défibrillateur)

Selon un mode de réalisation, en référence à la figure 1, le premier signal de détection S_{D} comprend un signal détecté par une électrode 2 d'un dispositif électrique 1. Le dispositif électrique 1 comprend par exemple un dispositif médical tel qu'un défibrillateur automatique implantable ou un « pacemaker ». Selon un autre exemple, le dispositif électrique 1 comprend un dispositif sous-cutané.

Le dispositif électrique 1 est par exemple implémenté dans le corps d'un patient. Dans un exemple, le dispositif électrique 1 est destiné à stimuler les muscles cardiaques. La stimulation est par exemple mise en œuvre lorsque le rythme du cœur du patient est considéré comme caractéristique d'une arythmie, par exemple en cas de tachycardie ventriculaire ou de fibrillation ventriculaire, ou encore en cas de bradycardie ventriculaire.

Selon plusieurs exemples, le dispositif électrique 1 comprend un défibrillateur automatique implantable à chambre unique, à deux chambres, ou encore à trois chambres. Le nombre de chambres indique le nombre de sondes qui relient le dispositif électrique 1 à des cavités cardiaques, par exemple aux ventricules et aux oreillettes. Un avantage est de stimuler un nombre donné de cavités cardiaques.

De manière plus générale, tout type de dispositif, implantable ou non et visant à mesurer ou stimuler l'activité cardiaque d'un patient est susceptible d'être utilisé pour la mise en œuvre du procédé selon l'invention.

Le dispositif électrique 1 nécessite une alimentation électrique pour fonctionner. Selon un exemple, la source d'alimentation électrique mise en œuvre pour alimenter le dispositif électrique 1 comprend une batterie. La batterie comprend par exemple une technologie Lithium-Ion. Toutefois, l'exemple précité n'est aucunement limitatif et tout type de technologie de batterie est susceptible d'être mise en œuvre pour alimenter le dispositif électrique 1.

Selon plusieurs exemples, le dispositif électrique 1 comprend divers composants. Selon un cas, le dispositif électrique 1 comprend un boîtier. Le boîtier comprend par exemple une pile, des capteurs, des circuits électroniques ou encore une mémoire pour enregistrer des données.

Dans un mode de réalisation, le dispositif électrique 1 comprend un élément d'accumulation d'énergie électrique. Le dispositif électrique 1 comprend par exemple un condensateur. Selon un exemple, une quantité d'énergie électrique définie est stockée dans l'élément d'accumulation d'énergie électrique. La quantité d'énergie électrique stockée est par exemple déchargée pour délivrer automatiquement un choc thérapeutique à un patient en réponse à la détection d'une arythmie cardiaque.

Dans un mode de réalisation, le dispositif électrique 1 comprend une interface de communication INT_{C.} Selon un cas, l'interface de communication INT_{C} est configurée pour échanger des données avec un équipement distant, tel qu'un équipement d'un réseau de données NET. L'interface de communication INT_{C} comprend par exemple un télétransmetteur. Selon un cas, l'interface de communication INT_{C} est paramétrée pour échanger des données automatiquement et à intervalles réguliers avec un équipement distant.

Selon un exemple de réalisation, l'interface de communication INT_{C} est configurée pour échanger des données avec un dispositif émetteur/récepteur. Dans un exemple, le dispositif émetteur/récepteur est configuré pour recevoir des données émises par l'interface de communication INT_{C} et pour transmettre automatiquement les données reçues vers un équipement distant. Un avantage est de pouvoir envoyer des données acquises par le dispositif vers un équipement distant, par exemple vers un centre de contrôle distant.

### Sonde(s)

Selon un mode de réalisation, en référence à la figure 1, le dispositif électrique 1 comprend une sonde 2. Dans un exemple, la sonde 2 est permet de relier le dispositif électrique 1 à une cavité cardiaque d'un patient.

Selon un cas, la sonde 2 comprend des composants électroniques. Les composants électroniques sont par exemple aptes à délivrer des impulsions électriques calibrées en fréquence et en amplitude.

Selon plusieurs exemples, la sonde 2 comprend divers matériaux et/ou alliages. Les alliages comprennent par exemple du titane et/ou du carbone. Toutefois, ces exemples ne sont pas limitatifs et la sonde 2 est susceptible de comprendre tout type de matériau ou d'alliages.

Selon d'autres exemples, d'autres sondes sont susceptibles d'être mises en œuvre pour mesurer l'activité électrique cardiaque, par exemple une électrode positionnée dans le ventricule gauche du patient. Plus généralement, tout type de sonde reliée au muscle cardiaque ou encore tout type de sonde extra cardiaque, par exemple reliée à un défibrillateur sous-cutané, est susceptible d'être mise en œuvre dans le cadre de l'invention.

Selon un mode de réalisation préféré, le dispositif électrique 1 comprend une unique sonde 2. La sonde 2 est par exemple positionnée dans le ventricule droit du cœur du patient. Ce cas est particulièrement avantageux en ce qu'une anomalie de l'électrode ventriculaire droite est susceptible d'avoir des conséquences dramatiques pouvant aller jusqu'au décès du patient. De plus, étant donné qu'à l'heure actuelle, la majorité des patients sont équipés d'une sonde positionnée dans le ventricule droit sans nécessairement être équipés d'une autre sonde complémentaire, l'invention trouve un intérêt particulier à être mise en œuvre à partir des signaux mesurés par une sonde unique positionnée dans le ventricule droit d'un patient.

Selon un autre exemple, la sonde est une électrode sous-cutanée.

Dans un mode de réalisation, le dispositif électrique 1 comprend une pluralité de sondes. Selon un exemple, le stimulateur cardiaque 1 comprend deux sondes implémentées chacune dans un ventricule du cœur du patient. Selon un autre exemple, le stimulateur cardiaque comprend trois sondes, la troisième sonde étant implémentée dans le sinus coronaire. Cela est particulièrement avantageux pour le traitement ou la prévention de certaines pathologies d'insuffisance cardiaque.

Toutefois, les exemples précités ne sont pas limitatifs. Plus généralement, tout dispositif électrique comprenant un nombre quelconque de sondes est susceptible d'être mis en œuvre dans le cadre de l'invention.

Selon un mode de réalisation, au moins une sonde 2 est bipolaire. La sonde 2 comprend par exemple un élément métallique. L'élément métallique est par exemple un ressort. Un avantage est de permettre la délivrance d'un choc électrique par le défibrillateur.

Dans un mode de réalisation, la sonde 2 comprend un matériau isolant. Selon un exemple, le matériau isolant comprend du polyuréthane et/ou du silicone. Toutefois, cet exemple est donné à titre indicatif et n'est pas limitatif. Plus généralement, la sonde 2 est susceptible de comprendre tout type de matériau isolant ou encore une combinaison de plusieurs matériaux isolants entre eux.

Selon un autre aspect, l'invention concerne un système configuré pour mettre en œuvre l'une quelconque des étapes du procédé selon l'invention.

Dans un mode de réalisation, le système comprend le dispositif électrique 1 comprenant la sonde 2.

Dans un mode de réalisation, le dispositif électrique 1 comprenant la sonde 2 est configuré pour générer le premier signal de détection S_{D}. Le premier signal de détection S_{D} est par exemple généré en réponse à la réception d'un courant électrique cardiaque par la sonde 2.

Dans un mode de réalisation, le système comprend une mémoire. La mémoire est configurée pour stocker des données. Les données comprennent par exemple des images acquises de l'électrogramme E_{G} ou encore l'information d'identification I_{id} associée au premier signal de détection S_{D}. Plus généralement, tout type d'information généré ou reçu par le système est susceptible d'être enregistré dans la mémoire.

Dans un mode de réalisation, le système comprend une interface de communication INT_{C}. Dans un exemple, l'interface de communication INT_{C} est configurée pour échanger des données avec au moins un autre dispositif. Selon un exemple, l'interface de communication INT_{C} est configurée pour échanger des données avec un équipement d'un réseau de données NET tel qu'un serveur distant. Par « échanger des données », on entend aussi bien que l'interface de communication « émet » des données vers un équipement distant que l'interface de communication INT_{C} « reçoit » des données émises par un équipement distant. Selon un exemple, les données émises par l'interface de communication INT_{C} comprennent des données enregistrées sur la mémoire du système. Selon un autre exemple, les données reçues par l'interface de communication sont enregistrées dans la mémoire du système.

Selon un autre aspect, l'invention concerne un produit programme d'ordinateur comprenant des instructions qui, lorsque le programme est exécuté par un ordinateur, conduisent celui-ci à mettre en œuvre l'une quelconque des étapes du procédé selon l'invention.

Selon un mode de réalisation, le programme est chargé sur un dispositif médical comprenant une pluralité de données enregistrées en mémoire. Selon un exemple, le dispositif médical sur lequel est chargé le programme comprend un défibrillateur automatique implantable. Dans un exemple, le programme met en œuvre les étapes du procédé de l'invention à partir des informations enregistrées dans le dispositif médical.

### Système

Selon un autre aspect, l'invention concerne un système pour générer une information d'identification I_{id} d'un signal de détection.

Dans un mode de réalisation, le système comprend au moins un instrument de mesure. Selon un cas, l'instrument de mesure comprend un instrument de mesure d'une activité électrique ; par exemple une électrode pour mesurer une activité électrique cardiaque.

Dans un mode de réalisation, l'instrument de mesure est configuré pour générer un signal en réponse à la réception d'un courant électrique cardiaque. Le signal généré comprend par exemple un signal de détection, tel que le premier signal de détection S_{D}.

Dans un mode de réalisation, le système comprend un calculateur K. Selon un cas, le calculateur K est configuré pour traiter des données. Les traitements effectués par le calculateur K sont par exemple mis en œuvre sur des données mesurées au moyen d'un instrument de mesure, tel qu'une électrode cardiaque. Dans un exemple, le traitement des données comprend la mise en œuvre d'opérations mathématiques sur lesdites données à traiter, par exemple des comparaisons de valeurs entre elles ou avec des valeurs seuil.

Dans un mode de réalisation, le calculateur K est configuré pour mettre en œuvre un algorithme. L'algorithme mis en œuvre comprend par exemple l'un des algorithmes de comparaison A_{COMP1}, A_{COMP2}.

Dans un mode de réalisation, le calculateur K est configuré pour mettre en œuvre une ou plusieurs fonctions. Les fonctions mises en œuvre par le calculateur K comprennent par exemple une fonction d'attribution. Dans un exemple, la fonction d'attribution mise en œuvre par le calculateur K comprend l'attribution de l'information d'identification I_{id} à un signal, par exemple au premier signal de détection S_{D}. L'information d'identification I_{id} est par exemple déterminée en fonction du résultat des comparaisons réalisées par un ou plusieurs algorithmes, par exemple un algorithme de comparaison ACOMP1, A_{COMP2}.

Dans un mode de réalisation, le système comprend un afficheur. Selon un cas, l'afficheur est configuré pour afficher une représentation graphique d'une mesure d'une donnée physique ou d'un ensemble de données physiques, par exemple un électrogramme E_{G} comportant un signal de détection tel que le premier signal de détection S_{D}.

Dans un mode de réalisation, l'afficheur est configuré pour générer un ou plusieurs marqueurs graphiques en superposition de l'électrogramme E_{G}. Les marqueurs graphiques comprennent par exemple des marqueurs temporels pour borner des intervalles temporels associés aux signaux de détection S_{D}, S_{D-1}, S_{D-2}, S_{D-3}. Les intervalles temporels comprennent par exemple les intervalles i, i-1 i-2, i-3.

En résumé, l'invention porte sur la comparaison des intervalles de temps associés à des signaux de détection avec des valeurs seuil pour déterminer si ces signaux sont caractéristiques d'un évènement physiologique ou si ces signaux sont caractéristiques d'un défaut matériel. L'invention est définie par les revendications annexées.

## Revendications

1. Procédé mis en œuvre par ordinateur pour attribuer une information d'identification (I_{id}) à un signal de détection (S_{D}) comprenant :
• Acquisition, au moyen d'une sonde, d'un premier signal de détection (S_{D}) en réponse à la réception d'un courant électrique cardiaque, ledit signal de détection (S_{D}) comportant une portion de signal correspondant à un motif électrique sur un premier intervalle de temps (i), ledit intervalle de temps étant défini entre deux évènements définis à partir d'au moins un motif électrique
• Mise en œuvre d'un premier algorithme de comparaison (A_{COMP1}) comprenant :
▪ Comparaison (COMP₁) du premier intervalle de temps (i) avec un premier seuil (Vₛₑᵤᵢₗ₁) ;
▪ Comparaison (COMP₂) d'un second intervalle de temps (i-1) associé à une portion d'un second signal de détection (S_{D-1}) antérieur au premier signal de détection (S_{D}) avec un second seuil (Vₛₑᵤᵢₗ₂) ;
• Attribution d'une information d'identification (I_{id}) au premier signal de détection (S_{D}), ladite information d'identification (I_{id}) étant déterminée en fonction du résultat des comparaisons réalisées par l'algorithme de comparaison (A_{comp1}) et ladite information d'identification (I_{id}) permettant de discriminer un signal de détection (S_{D}) ayant une cause physiologique d'un signal de détection (S_{D}) ayant pour cause un dysfonctionnement d'un matériel électronique.

2. Procédé selon la revendication 1, dans lequel le premier algorithme de comparaison (A_{COMP1}) comprend :
• Comparaison (COMP₃) d'un troisième intervalle de temps (i-2) associé à une portion d'un troisième signal de détection (S_{D-2}) avec un troisième seuil (Vₛₑᵤᵢₗ₃), ledit troisième signal de détection (S_{D-2}) étant antérieur au second signal de détection (S_{D-1}).

3. Procédé selon l'une quelconque des revendications 1 à 2, comprenant
• Mise en œuvre d'un second algorithme de comparaison (A_{COMP2}) comprenant :
▪ Comparaison (COMP₄) du premier intervalle de temps (i) avec un quatrième seuil (Vₛₑᵤᵢₗ₄) ;
▪ Comparaison (COMP₅) du second intervalle de temps (i-1) avec un cinquième seuil (Vₛₑᵤᵢₗ₅) ;
▪ Comparaison (COMP₅) du troisième intervalle de temps (i-2) avec un sixième seuil (Vₛₑᵤᵢₗ₆).

4. Procédé selon l'une quelconque des revendications 2 à 3, comprenant la mise en œuvre, par le premier algorithme de comparaison (A_{COMP1}) ou le second algorithme de comparaison (A_{COMP2}), de la comparaison (COMP₇) d'un septième seuil (Vₛₑᵤᵢₗ₇) avec un quatrième intervalle de temps (i-3) associé à une portion d'un quatrième signal de détection (S_{D-3}) antérieur au troisième signal de détection (S_{D-2}).

5. Procédé selon l'une quelconque des revendications 3 à 4, dans lequel l'information d'identification (I_{id}) est attribuée au premier signal de détection S_{D} en fonction du résultat des comparaisons d'au moins un des deux algorithmes de comparaison (A_{COMP1}, A_{COMP2}) ou d'une combinaison des deux algorithmes de comparaison (A_{COMP1}, A_{COMP2}).

6. Procédé selon l'une quelconque des revendications 4 à 5, dans lequel un ou plusieurs seuils parmi les seuils Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ et Vₛₑᵤᵢₗ₇ sont définis par :
▪ Une valeur de seuil prédéfinie ou ;
▪ Une valeur médiane ou une moyenne de plusieurs valeurs entre elles ou ;
▪ Une fonction mathématique définie par rapport un ou plusieurs intervalles de temps (i, i-1, i-2, i-3) ou ;
▪ Une fonction mathématique indépendante des valeurs des intervalles acquis ou ;
▪ Une combinaison de plusieurs fonctions mathématiques.

7. Procédé selon l'une quelconque des revendications précédentes, dans lequel l'information d'identification (I_{id}) attribuée au premier signal de détection (S_{D}) comprend soit :
• Une information physiologique caractérisant une donnée de rythme cardiaque ;
• Une information d'anomalie caractérisant une donnée d'un signal non physiologique.

8. Procédé selon la revendication 7, dans lequel l'information d'identification (I_{id}) attribuée au premier signal de détection (S_{D}) comprend une information d'anomalie caractéristique d'une rupture de sonde :
• Soit lorsque le premier intervalle de temps (i) est inférieur au premier seuil (Vₛₑᵤᵢₗ₁), le second intervalle de temps (i-1) est supérieur au second seuil (Vₛₑᵤᵢₗ₂) et le troisième intervalle de temps (i-2) est supérieur au troisième seuil (Vₛₑᵤᵢₗ₃) ;
• Soit lorsque le premier intervalle de temps (i) est inférieur au premier seuil (Vₛₑᵤᵢₗ₁) et le second intervalle de temps (i-1) est supérieur au second seuil (Vₛₑᵤᵢₗ₂), le troisième intervalle de temps (i-2) est supérieur au troisième seuil (Vₛₑᵤᵢₗ₃) et le quatrième intervalle de temps (i-3) est supérieur au septième seuil (Vₛₑᵤᵢₗ₇).

9. Procédé selon l'une quelconque des revendications 7 à 8, comprenant
• Génération d'une notification ou d'une alerte (A₁) lorsque l'information d'identification (I_{id}) attribuée au premier signal de détection (S_{D}) comprend l'information d'anomalie ;
• Enregistrement de ladite alerte/notification (A₁) au sein d'un espace mémoire,
• Emission de ladite alerte/notification (A₁) générée vers un équipement d'un réseau de données distant (NET).

10. Procédé selon l'une quelconque des revendications 4 à 9, comprenant :
• Génération d'un électrogramme (E_{G}) comprenant une représentation graphique d'au moins un signal de détection parmi les signaux (S_{D}, S_{D-1}, S_{D-2}, S_{D-3}) et d'au moins un intervalle de temps (i, i-1, i-2, i-3) ;
• Acquisition, enregistrement et transmission de l'image de l'électrogramme (E_{G}) acquise vers au moins un équipement d'un réseau de données (NET).

11. Système pour générer une information d'identification d'un signal de détection cardiaque comprenant :
• Un dispositif électrique (1) comportant au moins une sonde (2) pour acquérir un premier signal de détection (S_{D}) en réponse à la réception d'un courant électrique cardiaque, ledit signal de détection (S_{D}) comportant une portion de signal correspondant à un motif électrique sur un premier intervalle de temps (i) ;
• Un calculateur (K) configuré pour mettre en œuvre :
▪d'une part un algorithme de comparaison (A_{COMP1}) comprenant :
i. Comparaison (COMP₁) du premier intervalle de temps (i) avec un premier seuil (Vₛₑᵤᵢₗ₁) ;
ii. Comparaison (COMP₂) d'un second intervalle de temps (i-1) associé à une portion d'un second signal de détection (S_{D-1}) antérieur au premier signal de détection (S_{D}) avec un second seuil (Vₛₑᵤᵢₗ₂) ;
▪ d'autre part une fonction d'attribution d'une information d'identification (I_{id}) au premier signal de détection (S_{D}), ladite information d'identification (I_{id}) étant déterminée en fonction du résultat des comparaisons réalisées par l'algorithme de comparaison (A_{comp1}), ladite information d'identification (I_{id}) permettant de discriminer un signal de détection (S_{D}) ayant une cause physiologique d'un signal de détection (S_{D}) ayant pour cause un dysfonctionnement d'un matériel électronique.
• Un afficheur pour générer un marqueur graphique en superposition d'un électrogramme comportant le premier signal de détection (S_{D}) afin de localiser temporellement l'information d'identification (I_{id}) du premier signal de détection (S_{D}).
• Une mémoire (M) pour enregistrer des données ;
• Une interface de communication pour échanger des données avec un équipement d'un réseau de données distant (NET) ;

12. Produit programme d'ordinateur comprenant des instructions qui, lorsque le programme est exécuté par un ordinateur, conduisent celui-ci à mettre en œuvre les étapes suivantes :
• Mise en œuvre d'un premier algorithme de comparaison (A_{COMP1}) comprenant :
▪ Comparaison (COMP₁) d'un premier intervalle de temps (i), sur lequel s'étend un motif électrique d'une portion d'un premier signal de détection (S_{D}) acquis au moyen d'une sonde, avec un premier seuil (Vₛₑᵤᵢₗ₁) ;
▪ Comparaison (COMP₂) d'un second intervalle de temps (i-1) associé à une portion d'un second signal de détection (S_{D-1}) antérieur au premier signal de détection (S_{D}) avec un second seuil (Vₛₑᵤᵢₗ₂) ;
• Attribution d'une information d'identification (I_{id}) au premier signal de détection (S_{D}), ladite information d'identification (I_{id}) étant déterminée en fonction du résultat des comparaisons réalisées par l'algorithme de comparaison (A_{comp1}) et ladite information d'identification (I_{id}) permettant de discriminer un signal de détection (S_{D}) ayant une cause physiologique d'un signal de détection (S_{D}) ayant pour cause un dysfonctionnement d'un matériel électronique.

## Patentansprüche

1. Computerimplementiertes Verfahren zum Zuweisen einer Identifikationsinformation (I_{id}) zu einem Detektionssignal (S_{D}) umfassend:
• Erfassen eines ersten Detektionssignals (S_{D}) mittels einer Sonde als Reaktion auf den Empfang eines elektrischen Herzstroms, wobei das Detektionssignal (S_{D}) einen Signalabschnitt umfasst, der einem elektrischen Muster über ein erstes Zeitintervall (i) entspricht, wobei das Zeitintervall zwischen zwei Ereignissen definiert ist, die anhand mindestens eines elektrischen Musters definiert sind;
• Durchführen eines ersten Vergleichsalgorithmus (A_{COMP1}), umfassend:
▪ Vergleichen (COMP₁) des ersten Zeitintervalls (i) mit einem ersten Schwellenwert (Vₛₑᵤᵢₗ₁);
▪ Vergleichen (COMP₂) eines zweiten Zeitintervalls (i-1), das einem Abschnitt eines zweiten Detektionssignals (S_{D-1}) vor dem ersten Detektionssignal (S_{D}) zugeordnet ist, mit einem zweiten Schwellenwert (Vₛₑᵤᵢₗ₂);
• Zuweisen einer Identifikationsinformation (I_{id}) zum ersten Detektionssignal (S_{D}), wobei die Identifikationsinformation (I_{id}) in Abhängigkeit vom Ergebnis der vom Vergleichsalgorithmus (A_{comp1}) durchgeführten Vergleiche bestimmt wird und die Identifikationsinformation (I_{id}) es ermöglicht, ein Detektionssignal (S_{D}) mit physiologischer Ursache von einem Detektionssignal (S_{D}) mit einer Fehlfunktion einer elektronischen Vorrichtung als Ursache zu unterscheiden.

2. Verfahren nach Anspruch 1, wobei der erste Vergleichsalgorithmus (A_{COMP1}) umfasst:
• Vergleichen (COMP₃) eines dritten Zeitintervalls (i-2), das einem Abschnitt eines dritten Detektionssignals (S_{D-2}) zugeordnet ist, mit einem dritten Schwellenwert (Vₛₑᵤᵢₗ₃), wobei das dritte Detektionssignal (S_{D-2}) vor dem zweiten Detektionssignal (S_{D-1}) liegt.

3. Verfahren nach einem der Ansprüche 1 bis 2, umfassend:
• Durchführen eines zweiten Vergleichsalgorithmus (A_{COMP2}), umfassend:
▪ Vergleichen (COMP₄) des ersten Zeitintervalls (i) mit einem vierten Schwellenwert (Vₛₑᵤᵢₗ₄);
▪ Vergleichen (COMP₅) des zweiten Zeitintervalls (i-1) mit einem fünften Schwellenwert (Vₛₑᵤᵢₗ₅);
▪ Vergleichen (COMP ₆) des dritten Zeitintervalls (i-2) mit einem sechsten Schwellenwert (Vₛₑᵤᵢₗ₆).

4. Verfahren nach einem der Ansprüche 2 bis 3, umfassend das Durchführen, durch den ersten Vergleichsalgorithmus (A_{COMP1}) oder den zweiten Vergleichsalgorithmus (A_{COMP2}), des Vergleichs (COMP₇) eines siebten Schwellenwerts (Vₛₑᵤᵢₗ₇) mit einem vierten Zeitintervall (i-3), das einem Abschnitt eines vierten Detektionssignals (S_{D-3}) zugeordnet ist, das vor dem dritten Detektionssignal (S_{D-2}) liegt.

5. Verfahren nach einem der Ansprüche 3 bis 4, wobei die Identifikationsinformation (I_{id}) dem ersten Detektionssignal S_{D} in Abhängigkeit vom Ergebnis der Vergleiche mindestens eines der beiden Vergleichsalgorithmen (A_{COMP1}, A_{COMP2}) oder einer Kombination der beiden Vergleichsalgorithmen (A_{COMP1}, A_{COMP2}) zugewiesen wird.

6. Verfahren nach einem der Ansprüche 4 bis 5, wobei einer oder mehrere der Schwellenwerte Vₛₑᵤᵢₗ₁, Vₛₑᵤᵢₗ₂, Vₛₑᵤᵢₗ₃, Vₛₑᵤᵢₗ₄, V_{seuil5,} Vₛₑᵤᵢₗ₆ und Vₛₑᵤᵢₗ₇ definiert sind durch:
▪ einen vordefinierten Schwellenwert oder;
▪ einen Medianwert oder einen Durchschnittswert mehrerer Werte untereinander oder;
▪ eine mathematische Funktion, die in Bezug auf ein oder mehrere Zeitintervalle (i, i-1, i-2, i-3) definiert ist, oder;
▪ eine mathematische Funktion, die unabhängig von den erfassten Intervallwerten ist, oder
▪ eine Kombination aus mehreren mathematischen Funktionen.

7. Verfahren nach einem der vorstehenden Ansprüche, wobei die dem ersten Detektionssignal (S_{D}) zugewiesene Identifikationsinformation (I_{id}) eines umfasst von:
• einer physiologischen Information, die eine Herzfrequenzangabe charakterisiert;
• einer Anomalieinformation, die eine nicht physiologische Signaldatenangabe charakterisiert.

8. Verfahren nach Anspruch 7, wobei die dem ersten Detektionssignal (S_{D}) zugewiesene Identifikationsinformation (I_{id}) eine für einen Sondenbruch charakteristische Anomalieinformation umfasst:
• entweder, wenn das erste Zeitintervall (i) kleiner als der erste Schwellenwert (Vₛₑᵤᵢₗ₁) ist, das zweite Zeitintervall (i-1) größer als der zweite Schwellenwert (Vₛₑᵤᵢₗ₂) ist und das dritte Zeitintervall (i-2) größer als der dritte Schwellenwert (Vₛₑᵤᵢₗ₃) ist;
• oder, wenn das erste Zeitintervall (i) kleiner als der erste Schwellenwert (Vₛₑᵤᵢₗ₁) ist und das zweite Zeitintervall (i-1) größer als der zweite Schwellenwert (Vₛₑᵤᵢₗ₂) ist, das dritte Zeitintervall (i-2) größer als der dritte Schwellenwert (Vₛₑᵤᵢₗ₃) ist und das vierte Zeitintervall (i-3) größer als der siebte Schwellenwert (Vₛₑᵤᵢₗ₇) ist.

9. Verfahren nach einem der Ansprüche 7 bis 8, umfassend:
• Erzeugen einer Benachrichtigung oder eines Alarms (A₁), wenn die dem ersten Detektionssignal (S_{D}) zugewiesene Identifikationsinformation (I_{id}) die Anomalieinformation enthält;
• Aufzeichnen des Alarms/der Benachrichtigung (A₁) in einem Speicherbereich,
• Senden des erzeugten Alarms/der Benachrichtigung (A₁) an ein Gerät eines entfernten Datennetzwerks (NET).

10. Verfahren nach einem der Ansprüche 4 bis 9, umfassend:
• Erzeugen eines Elektrogramms (E_{G}), das eine grafische Darstellung mindestens eines Detektionssignals aus den Signalen (S_{D}, S_{D-1}, S_{D-2}, S_{D-3}) und mindestens eines Zeitintervalls (i, i-1, i-2, i-3) umfasst;
• Erfassen, Aufzeichnen und Übertragen des erfassten Elektrogrammbildes (E_{G}) an mindestens ein Gerät eines Datennetzwerks (NET).

11. System zum Erzeugen einer Identifikationsinformation eines Herzdetektionssignals, umfassend:
• eine elektrische Vorrichtung (1) mit mindestens einer Sonde (2) zum Erfassen eines ersten Detektionssignals (S_{D}) als Reaktion auf den Empfang eines elektrischen Herzstroms, wobei das Detektionssignal (S_{D}) einen Signalabschnitt umfasst, der einem elektrischen Muster über ein erstes Zeitintervall (i) entspricht;
• einen Rechner (K), der ausgelegt ist zum Durchführen von:
▪ einerseits einem Vergleichsalgorithmus (A_{COMP1}), umfassend:
i. Vergleichen (COMP₁) des ersten Zeitintervalls (i) mit einem ersten Schwellenwert (Vₛₑᵤᵢₗ₁);
ii. Vergleichen (COMP₂) eines zweiten Zeitintervalls (i-1), das einem Abschnitt eines zweiten Detektionssignals (S_{D-1}) vor dem ersten Detektionssignal (S_{D}) zugeordnet ist, mit einem zweiten Schwellenwert (Vₛₑᵤᵢₗ₂);
▪ andererseits einer Funktion zum Zuweisen einer Identifikationsinformation (I_{id}) zum ersten Detektionssignal (S_{D}), wobei die Identifikationsinformation (I_{id}) in Abhängigkeit vom Ergebnis der vom Vergleichsalgorithmus (A_{comp1}) durchgeführten Vergleiche bestimmt wird, wobei die Identifikationsinformation (I_{id}) es ermöglicht, ein Detektionssignal (S_{D}) mit physiologischer Ursache von einem Detektionssignal (S_{D}) mit einer Fehlfunktion einer elektronischen Vorrichtung als Ursache zu unterscheiden;
• eine Anzeige zum Erzeugen einer grafischen Markierung, die ein Elektrogramm überlagert, das das erste Detektionssignal (S_{D}) umfasst, um die Identifikationsinformation (I_{id}) des ersten Detektionssignals (S_{D}) zeitlich zu lokalisieren;
• einen Speicher (M) zum Aufzeichnen von Daten;
• eine Kommunikationsschnittstelle zum Austauschen von Daten mit einem Gerät eines entfernten Datennetzwerks (NET).

12. Computerprogrammprodukt mit Anweisungen, die, wenn das Programm von einem Computer ausgeführt wird, diesen dazu veranlassen, die folgenden Schritte durchzuführen:
• Durchführen eines ersten Vergleichsalgorithmus (A_{COMP1}), umfassend:
▪ Vergleichen (COMP₁) eines ersten Zeitintervalls (i), über das sich ein elektrisches Muster eines Abschnitts eines ersten Detektionssignals (S_{D}) erstreckt, das mittels einer Sonde erfasst wurde, mit einem ersten Schwellenwert (Vₛₑᵤᵢₗ₁);
▪ Vergleichen (COMP₂) eines zweiten Zeitintervalls (i-1), das einem Abschnitt eines zweiten Detektionssignals (S_{D-1}) vor dem ersten Detektionssignal (S_{D}) zugeordnet ist, mit einem zweiten Schwellenwert (Vₛₑᵤᵢₗ₂);
• Zuweisen einer Identifikationsinformation (l_{id}) zum ersten Detektionssignal (S_{D}), wobei die Identifikationsinformation (l_{id}) in Abhängigkeit vom Ergebnis der vom Vergleichsalgorithmus (A_{comp1}) durchgeführten Vergleiche bestimmt wird und die Identifikationsinformation (l_{id}) es ermöglicht, ein Detektionssignal (S_{D}) mit physiologischer Ursache von einem Detektionssignal (S_{D}) mit einer Fehlfunktion einer elektronischen Vorrichtung als Ursache zu unterscheiden.

## Claims

1. A computer-implemented method for assigning an item of identification information (I_{id}) to a detection signal (S_{D}), comprising:
• acquiring, by means of a probe, a first detection signal (S_{D}) in response to receiving a cardiac electrical current, said detection signal (S_{D}) comprising a signal portion corresponding to an electrical pattern over a first time interval (i), said time interval being defined between two events defined from at least one electrical pattern;
• implementing a first comparison algorithm (A_{COMP1}) comprising:
▪ comparing (COMP₁) the first time interval (i) with a first threshold (Vₛₑᵤᵢₗ₁);
▪ comparing (COMP₂) a second time interval (i-1) associated with a portion of a second detection signal (S_{D-1}) prior to the first detection signal (S_{D}) with a second threshold (Vₛₑᵤᵢₗ₂);
• assigning an item of identification information (I_{id}) to the first detection signal (S_{D}), said item of identification information (I_{id}) being determined on the basis of the result of the comparisons performed by the comparison algorithm (A_{COMP1}) and said item of identification information (I_{id}) making it possible to discriminate a detection signal (S_{D}) having a physiological cause from a detection signal (S_{D}) having a malfunction of electronic hardware as its cause.

2. The method according to claim 1, wherein the first comparison algorithm (A_{COMP1}) comprises:
• comparing (COMP₃) a third time interval (i-2) associated with a portion of a third detection signal (S_{D-2}) with a third threshold (Vₛₑᵤᵢₗ₃), said third detection signal (S_{D-2}) being prior to the second detection signal (S_{D-1}).

3. The method according to any one of claims 1 to 2, comprising:
• implementing a second comparison algorithm (A_{COMP2}) comprising:
▪ comparing (COMP₄) the first time interval (i) with a fourth threshold (Vₛₑᵤᵢₗ₄);
▪ comparing (COMP₅) the second time interval (i-1) with a fifth threshold (Vₛₑᵤᵢₗ₅);
▪ comparing (COMP₆) the third time interval (i-2) with a sixth threshold (Vₛₑᵤᵢₗ₆).

4. The method according to any one of claims 2 to 3, comprising: implementing, by means of the first comparison algorithm (A_{COMP1}) or the second comparison algorithm (A_{COMP2}), the comparison (COMP₇) of a seventh threshold (Vₛₑᵤᵢₗ₇) with a fourth time interval (i-3) associated with a portion of a fourth detection signal (S_{D-3}) prior to the third detection signal (S_{D-2}).

5. The method according to any one of claims 3 to 4, wherein the item of identification information (I_{id}) is assigned to the first detection signal S_{D} on the basis of the result of the comparisons of at least one of the two comparison algorithms (A_{COMP1}, A_{COMP2}) or a combination of the two comparison algorithms (A_{COMP1}, A_{COMP2}).

6. The method according to any one of claims 4 to 5, wherein one or more thresholds of the thresholds Vₛₑᵤᵢₗ₁, Vₛₑᵤᵢₗ₂, Vₛₑᵤᵢₗ₃, Vₛₑᵤᵢₗ₄, Vₛₑᵤᵢₗ₅, Vₛₑᵤᵢₗ₆ and Vₛₑᵤᵢₗ₇ are defined by:
▪ a predefined threshold value, or
▪ a median value or an average of several values together, or
▪ a mathematical function defined relative to one or more time intervals (i, i-1, i-2, i-3), or
▪ a mathematical function independent of the values of the acquired intervals, or
▪ a combination of several mathematical functions.

7. The method according to any one of the preceding claims, wherein the item of identification information (I_{id}) assigned to the first detection signal (S_{D}) comprises either:
• an item of physiological information characterizing an item of heart rate data;
• an item of anomaly information characterizing an item of non-physiological signal data.

8. The method according to claim 7, wherein the item of identification information (I_{id}) assigned to the first detection signal (S_{D}) comprises an item of anomaly information characteristic of a probe breakage:
• either when the first time interval (i) is below the first threshold (Vₛₑᵤᵢₗ₁), the second time interval (i-1) is above the second threshold (Vₛₑᵤᵢₗ₂) and the third time interval (i-2) is above the third threshold (Vₛₑᵤᵢₗ₃),
• or when the first time interval (i) is below the first threshold (Vₛₑᵤᵢₗ₁) and the second time interval (i-1) is above the second threshold (Vₛₑᵤᵢₗ₂), the third time interval (i-2) is above the third threshold (Vₛₑᵤᵢₗ₃) and the fourth time interval (i-3) is above the seventh threshold (Vₛₑᵤᵢₗ₇).

9. The method according to any one of claims 7 to 8, comprising:
• generating a notification or alert (A₁) when the item of identification information (I_{id}) assigned to the first detection signal (S_{D}) comprises the item of anomaly information;
• saving said alert/notification (A₁) in a memory space,
• emitting said generated alert/notification (A₁) to equipment of a remote data network (NET).

10. The method according to any one of claims 4 to 9, comprising:
• generating an electrogram (E_{G}) comprising a graphical representation of at least one detection signal of the signals (S_{D}, S_{D-1}, S_{D-2}, S_{D-3}) and of at least one time interval (i, i-1, i-2, i-3);
• acquiring, recording and transmitting the acquired image of the electrogram (E_{G}) to at least one item of equipment in a data network (NET).

11. A system for generating an item of identification information of a cardiac detection signal, comprising:
• an electrical device (1) comprising at least one probe (2) for acquiring a first detection signal (S_{D}) in response to receiving a cardiac electrical current, said detection signal (S_{D}) comprising a signal portion corresponding to an electrical pattern over a first time interval (i);
• a calculator (K) configured to implement:
• on the one hand, a comparison algorithm (A_{COMP1}) comprising:
i. comparing (COMP₁) the first time interval (i) with a first threshold (Vₛₑᵤᵢₗ₁);
ii. comparing (COMP₂) a second time interval (i-1) associated with a portion of a second detection signal (S_{D-1}) prior to the first detection signal (S_{D}) with a second threshold (Vₛₑᵤᵢₗ₂);
▪ on the other hand, a function for assigning an item of identification information (I_{id}) to the first detection signal (S_{D}), said item of identification information (I_{id}) being determined on the basis of the result of the comparisons performed by the comparison algorithm (A_{COMP1}), said item of identification information (I_{id}) making it possible to discriminate a detection signal (S_{D}) having a physiological cause from a detection signal (S_{D}) having a malfunction of electronic hardware as its cause,
• a display for generating a graphical marker overlaid on an electrogram comprising the first detection signal (S_{D}) in order to temporally locate the item of identification information (I_{id}) of the first detection signal(S_{D}).
• a memory (M) for recording data;
• a communication interface for exchanging data with equipment in a remote data network (NET);

12. A computer program product comprising instructions which, when the program is executed by a computer, cause said computer to implement the following steps:
• implementing a first comparison algorithm (A_{COMP1}) comprising:
▪ comparing (COMP₁) a first time interval (i), over which an electrical pattern of a portion of a first detection signal (S_{D}) acquired by means of a probe extends, with a first threshold (Vₛₑᵤᵢₗ₂);
▪ comparing (COMP₂) a second time interval (i-1) associated with a portion of a second detection signal (S_{D-1}) prior to the first detection signal (S_{D}) with a second threshold (Vₛₑᵤᵢₗ₂);
• assigning an item of identification information (I_{id}) to the first detection signal (S_{D}), said item of identification information (I_{id}) being determined on the basis of the result of the comparisons performed by the comparison algorithm (A_{COMP1}) and said item of identification information (I_{id}) making it possible to discriminate a detection signal (S_{D}) having a physiological cause from a detection signal (S_{D}) having a malfunction of electronic hardware as its cause.
